# FASCICULE DE BREVET EUROPEEN

(11) **EP 0 632 026 B1**
(45) Date de publication et mention de la délivrance du brevet: **19.11.1997**
(21) Numéro de dépôt: 94401267.3
(22) Date de dépôt: 08.06.1994
(51) Int. Cl.: C07D 213/75, A61K 31/44, C07F 5/02

(54) **Nouveaux dérivés d'alpha amino acides leur procédé de préparation et les compositions pharmaceutiques qui les contiennent**
Alpha Aminosäurederivate, Verfahren zu ihrer Herstellung und diese enthaltende pharmazeutische Zusammensetzungen
Alpha amino acid derivatives, a method for their preparation and pharmaceutical preparations containing them

(30) Priorité: 30.06.1993 FR 9307928
(43) Date de publication de la demande: 04.01.1995
(73) Titulaire: ADIR ET COMPAGNIE, 92415 Courbevoie Cédex (FR)
(72) Inventeur: Duflos, Muriel, F-44680 Saint Pazanne (FR); Robert, Piessard, Sylvie, F-44200 Nantes (FR); Welin, Lucien, F-44200 Nantes (FR); Le Baut, Guillaume, F-44230 St. Sebastien sur Loire (FR); Caignard, Daniel-Henri, F-75013 Paris (FR); Renard, Pierre, F-78000 Versailles (FR); Adam, Gérard, F-78600 Le Mesnil le Roi (FR)

(56) Documents cités:
- EP-A- 0 217 286
- CHEMICAL ABSTRACTS, vol. 79, no. 13, 1973, Columbus, Ohio, US; abstract no. 78046v, M.T. COCCO ET AL. page 435-436 ;

## Description

La présente invention concerne de nouveaux dérivés d'α amino acides, leur procédé de préparation et les compositions pharmaceutiques qui les contiennent.

De très nombreux dérivés d'acides aminés sont déjà connus.

La demanderesse a présentement découvert que les dérivés résultant du couplage d'un acide α aminé protégé, ou non, avec la 2-amino 4,6-diméthyl pyridine permettait l'obtention de dérivés totalement atoxiques et doués de propriétés antiinflammatoires ou/et hypolipémiantes de haut niveau.

Plus spécifiquement l'invention concerne les dérivés de formule générale (I) : 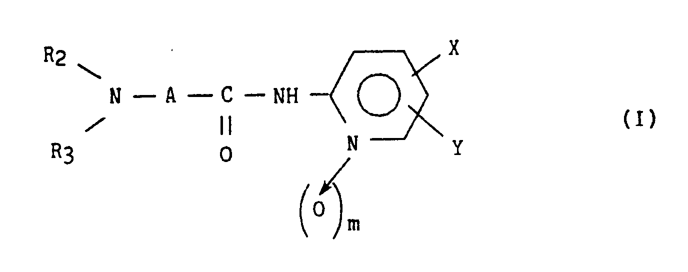 dans laquelle :
m représente 0 ou 1,
X et Y identiques ou différents représentent chacun un groupement alkyle,
A représente :
1) - un groupement 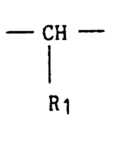
et R₁ représente :
   - un atome d'hydrogène,
   - un groupement alkyle,
   - un groupement alkyle substitué par un groupement aminé,
   - un groupement alkylthioalkyle,
   - un groupement alkyle substitué par un groupement aminé lui-même substitué sur le groupe amino par un groupement choisi parmi tert butoxy carbonyle, benzyloxy carbonyle, fluorénylméthyloxy carbonyle,
   - un groupement phényle ou phényl alkyle,
   - un groupement phényle ou phényl alkyle substitué sur le noyau aromatique par un groupement alkyle, hydroxyle, alkoxy, trifluorométhyle ou par un atome d'halogène,
   et dans ce cas - où A représente un groupement -CH(R₁)- :
R₂ représente un atome d'hydrogène ou R₂ forme avec R₁ et le chaînon CH-N qui les porte, un système carboné mono ou bicyclique renfermant le chaînon -CH-N, chaque cycle comprenant de 5 ou 6 sommets et pouvant être saturé ou non,
   et alors,
R₃ représente :
   - un atome d'hydrogène,
   - un groupement t.butoxycarbonyle, benzyloxycarbonyle, fluorénylméthyloxycarbonyle,
   - un groupement benzoyle,
   - un groupement benzoyle substitué par un groupement alkyle, alkoxy, hydroxyle, trifluorométhyle ou par un atome d'halogène,
   - un groupement 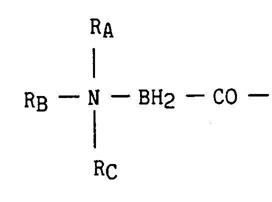 avec R_{A}, R_{B}, R_{C} identiques ou
      différents représentant un atome d'hydrogène ou un groupement alkyle, ou encore deux groupements parmi R_{A}, R_{B}, R_{C} forment avec l'atome d'azote qui les porte un système hétérocyclique choisi parmi pyrrolidine, pipéridine, azépine ou morpholine, le troisième groupement parmi R_{A}, R_{B} et R_{C} étant alors soit un atome d'hydrogène, soit un groupement alkyle,
   - un groupement 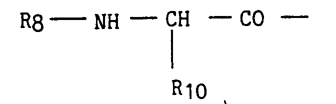
avec R₈ représentant hydrogène, t.butoxycarbonyle, benzyloxycarbonyle, fluorényl méthyloxycarbonyle, glycyle, glycyle substitué par un groupement t. butoxycarbonyle, benzyloxycarbonyle ou fluorénylméthyloxycarbonyle et R₁₀ représentant hydrogène ; alkyle ; alkyle substitué par un groupement amino ; alkyle substitué par un groupement amino lui-même substitué par un groupement t.butoxycarbonyle, benzyloxycarbonyle, fluorényl méthyloxycarbonyle ; un groupement phényle ou phénylalkyle ; un groupement phényle ou phénylalkyle substitué sur le noyau phényle par un groupement alkyle, hydroxyle, alkoxy, trifluorométhyle ou par un atome d'halogène,
2) - un groupement 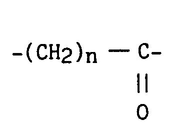 le groupement 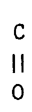 du radical A étant lié au groupement 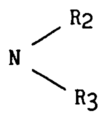 du dérivé de formule (I) avec n entier compris inclusivement entre 1 et 6,
et dans ce cas R₃ représente un groupement 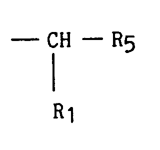
R₁ et R₂ conservant la définition donnée précédemment, et R₅ représentant un groupement B(OH)₂, carboxyle, alkoxy carbonyle, ou bien 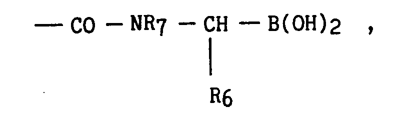
avec R₆ et R₁ identiques ou différents et ayant chacun la définition de R₁ telle que donnée préalablement, R₇ ayant la définition de R₂ donnée précédemment et étant identique à R₂ ou différent,
   leurs isomères, épimères, énantiomères, diastéréoisomères ainsi que leurs sels d'addition à un acide ou une base pharmaceutiquement acceptable, étant entendu que par alkyle ou par alkoxy on entend des groupements linéaires ou ramifiés comprenant de 1 à 6 atomes de carbone.

Parmi les acides pharmaceutiquement acceptables que l'on peut ajouter aux composés de formule (I) pour obtenir un sel, on peut citer à titre non limitatif les acides chlorhydrique, sulfurique, tartrique, maléique, fumarique, oxalique, méthane-sulfonique, camphorique etc...

Parmi les bases pharmaceutiquement acceptables que l'on peut ajouter aux composés de formule (I) renfermant un groupement acide pour obtenir un sel, on peut citer, à titre non limitatif les hydroxydes de sodium, potassium, calcium, ou magnesium, des carbonates de métaux alcalins ou alcalino terreux, ou des bases organiques comme la triéthylamine, la benzylamine, la diéthanolamine, la tert butylamine, la dicyclohexylamine, l'arginine, la lysine etc...

Des cas particuliers de l'invention concernent :
* les composés ayant la formule générale (I) : 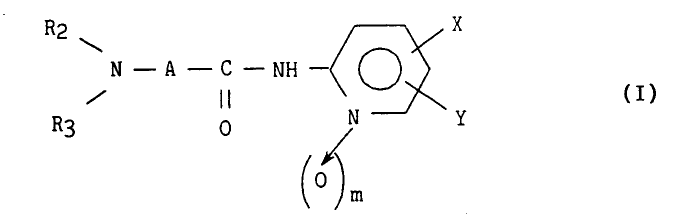 dans laquelle :
   m représente 0 ou 1,
   X et Y identiques ou différent représentent chacun un groupement alkyle,
   et A représente un groupement 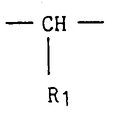
   et R₁ représente :
      - un atome d'hydrogène,
      - un groupement alkyle,
      - un groupement alkyle substitué par un groupement aminé,
      - un groupement alkylthioalkyle,
      - un groupement alkyle substitué par un groupement aminé lui-même substitué sur le groupe amino par un groupement choisi parmi tert butoxy carbonyle, benzyloxy carbonyle, fluorénylméthyloxy carbonyle,
      - un groupement phényle ou phényl alkyle,
      - un groupement phényle ou phényl alkyle substitué sur le noyau aromatique par un groupement alkyle, hydroxyle, alkoxy, trifluorométhyle ou par un atome d'halogène,
   R₂ représente un atome d'hydrogène ou R₂ forme avec R₁ et le chaînon CH-N- qui les porte, un système carboné mono ou bicyclique renfermant le chaînon -CH-N-, chaque cycle comprenant de 5 ou 6 sommets et pouvant être saturé ou non,
      et,
   R₃ représente :
      - un atome d'hydrogène,
      - un groupement t.butoxycarbonyle, benzyloxycarbonyle, fluorénylméthyloxycarbonyle,
      - un groupement benzoyle,
      - un groupement benzoyle substitué par un groupement alkyle, alkoxy, hydroxyle, trifluorométhyle ou par un atome d'halogène,
      - un groupement 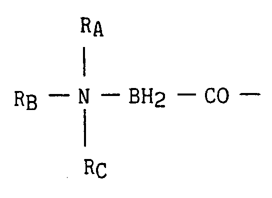 avec R_{A}, R_{B}, R_{C} identiques ou
         différents représentant un atome d'hydrogène ou un groupement alkyle, ou encore deux groupements parmi R_{A}, R_{B}, R_{C} forment avec l'atome d'azote qui les porte un système hétérocyclique choisi parmi pyrrolidine, pipéridine, azépine ou morpholine, le troisième groupement parmi R_{A}, R_{B} et R_{C} étant alors soit un atome d'hydrogène, soit un groupement alkyle,
         leurs isomères, épimères, énantiomères, diastéréoisomères ainsi que leurs sels d'addition à un acide ou une base pharmaceutiquement acceptable,
         étant entendu que par alkyle ou par alkoxy on entend des groupements linéaires ou ramifiés comprenant de 1 à 6 atomes de carbone.
* les composés de formule générale (I) : 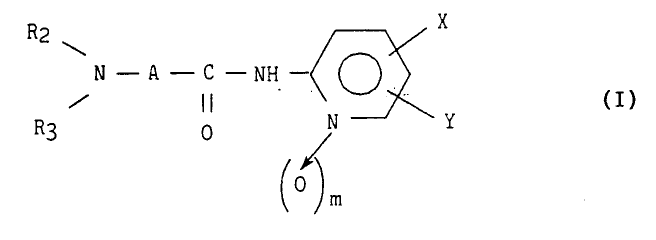 dans laquelle :
   m représente 0 ou 1,
   X et Y identiques ou différents représentent chacun un groupement alkyle
   et A représente un groupement 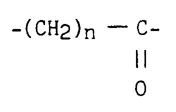 le groupement 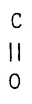 du radical A étant lié au groupement 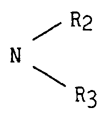 du dérivé de formule (I) avec n entier compris inclusivement entre 1 et 6,
   R₃ représente un groupement 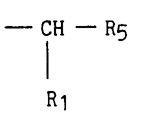
   et R₁ représente :
      - un atome d'hydrogène,
      - un groupement alkyle,
      - un groupement alkyle substitué par un groupement aminé,
      - un groupement alkylthioalkyle,
      - un groupement alkyle substitué par un groupement aminé lui-même substitué sur le groupe amino par un groupement choisi parmi tert butoxy carbonyle, benzyloxy carbonyle, fluorénylméthyloxy carbonyle,
      - un groupement phényle ou phényl alkyle,
      - un groupement phényle ou phényl alkyle substitué sur le noyau aromatique par un groupement alkyle, hydroxyle, alkoxy, trifluorométhyle ou par un atome d'halogène,
   R₂ représentant un atome d'hydrogène ou R₂ forme avec R₁ et le chaînon CH-NR₃ qui les porte, un système carboné mono ou bicyclique renfermant le chaînon -CH-N(R₃), chaque cycle comprenant de 5 ou 6 sommets et pouvant être saturé ou non,
   avec R₅ représentant un groupement B(OH)₂, carboxyle, alkoxy carbonyle, ou bien 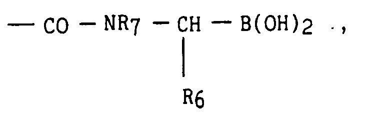
   avec R₆ et R₁ identiques ou différents et ayant chacun la définition de R₁ telle que donnée préalablement, R₇ ayant la définition de R₂ donnée précédemment et étant identique à R₂ ou différent,
      leurs isomères, épimères, énantiomères, diastéréoisomères ainsi que leurs sels d'addition à un acide ou une base pharmaceutiquement acceptable,
* les composés de formule générale I : 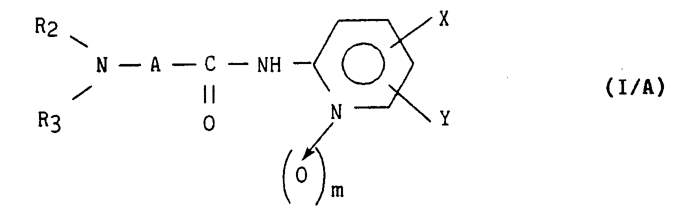
   dans laquelle m représente 0 ou 1 et X et Y identiques ou différents représentent chacun un groupement alkyle et A représente un groupement 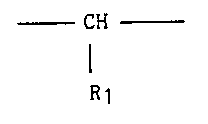
   et R₁ représente :
      - un atome d'hydrogène,
      - un groupement alkyle substitué par un groupement aminé,
      - un groupement alkylthioalkyle,
      - un groupement alkyle substitué par un groupement aminé lui-même substitué sur le groupe amino par un groupement choisi parmi tert butoxy carbonyle, benzyloxy carbonyle, fluorénylméthyloxy carbonyle,
      - un groupement phényle ou phényl alkyle,
      - un groupement phényle ou phényl alkyle substitué sur le noyau aromatique par un groupement alkyle, hydroxyle, alkoxy, trifluorométhyle ou par un atome d'halogène,
   et R₃ représente :
      - un groupement 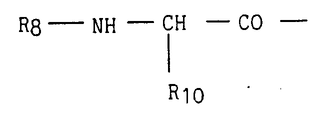
   avec R₈ représentant hydrogène, t.butoxycarbonyle, benzyloxycarbonyle, fluorényl méthyloxycarbonyle, glycyle, glycyle substitué par un groupement t.butoxycarbonyle benzyloxycarbonyle, fluorénylméthyloxycarbonyle et R₁₀ représentant hydrogène ; alkyle ; alkyle substitué par un groupement amino ; alkyle substitué par un groupement amino lui-même substitué par un groupement t.butoxycarbonyle, benzyloxycarbonyle, fluorényl méthyloxycarbonyle ; un groupement phényle, ou phénylalkyle, un groupement phényle ou phénylalkyle substitué sur le noyau phényle par un groupement alkyle, hydroxyle, alkoxy, trifluorométhyle ou par un atome d'halogène, leurs isomères, épimères, énantiomères, diastéréoisomères ainsi que leurs sels d'addition à un acide ou une base pharmaceutiquement acceptable,
* les composés de formule (I/A) : 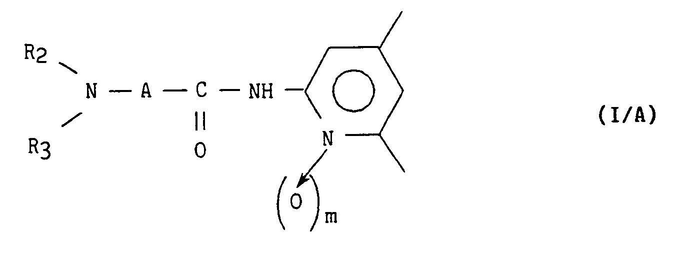
   avec m représentant 0 ou 1 et A représentant un groupement 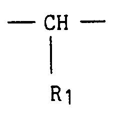 et
   R₁ choisi parmi hydrogène alkyle, benzyle, R₂ représente hydrogène, benzyloxy carbonyle, t.butoxy carbonyle et R₃ représente : 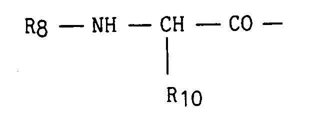
   avec R₈ représentant hydrogène, t.butoxycarbonyle, benzyloxycarbonyle, fluorényl méthyloxycarbonyle et R₁₀ représentant hydrogène ; alkyle ; alkyle substitué par un groupement amino ; alkyle substitué par un groupement amino lui-même substitué par un groupement t.butoxycarbonyle, benzyloxycarbonyle, fluorényl méthyloxycarbonyle ; un groupement phényle ou phénylalkyle, un groupement phényle ou phénylalkyle substitué sur le noyau phényle par un groupement alkyle, hydroxyle, alkoxy, trifluorométhyle ou par un atome d'halogène,
   leurs isomères, épimères, énantiomères, diastéréoisomères ainsi que leurs sels d'addition à un acide ou une base pharmaceutiquement acceptable,
* les composés de formule (I/A) 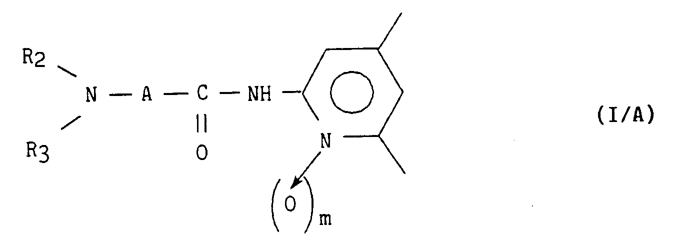
   avec m représentant 0 ou 1 et A représentant un groupement 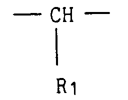 et
   R₁ choisi parmi hydrogène, alkyle, benzyle ou R₂ représente hydrogène et R₃ un groupement 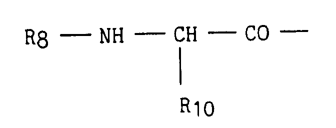
   avec R₈ représentant hydrogène, t.butoxycarbonyle, benzyloxycarbonyle, fluorényl méthylcarbonyle, glycyle, glycyle substitué par un groupement t.butoxycarbonyle, benzyloxycarbonyle, fluorénylméthyloxycarbonyle et R₁₀ représentant hydrogène ; alkyle ; alkyle substitué par un groupement amino ; alkyle substitué par un groupement amino lui-même substitué par un groupement t.butoxycarbonyle, benzyloxycarbonyle, fluorényl méthyloxycarbonyle ; un groupement phényle ou phénylalkyle, un groupement phényle ou phénylalkyle substitué sur le noyau phényle par un groupement alkyle, hydroxyle, alkoxy, trifluorométhyle ou par un atome d'halogène,
      leurs isomères, épimères, énantiomères, diastéréoisomères ainsi que leurs sels d'addition à un acide ou une base pharmaceutiquement acceptable,
* le composé qui est le N-2-benzyloxycarbonyle N(4,6-diméthylpyridin-2-yl) prolinamide ses isomères, ainsi que son N oxyde, ses sels d'addition à un acide ou une base pharmaceutiquement acceptable,
* le composé qui est le N-2-benzyloxycarbonyle-N-(4,6-diméthylpyridin-2-yl)glycinamide, ainsi que son N oxyde, ses sels d'addition à un acide ou une base pharmaceutiquement acceptable,
* le composé qui est le N-2-(3-fluorobenzoyl) N-(4,6-diméthyl pyridin-2 yl) glycinamide, ainsi que son N oxyde, ses sels d'addition à un acide ou une base pharmaceutiquement acceptable,
* les composés pour lesquels A représente un groupement : 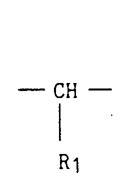 et R₃ un groupement 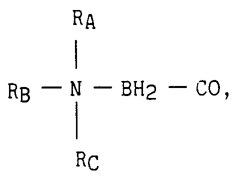 avec R_{A}, R_{B}, R_{C}
   identiques ou différents représentant un atome d'hydrogène ou un groupement alkyle, ou encore deux groupements parmi R_{A}, R_{B}, R_{C} forment avec l'atome d'azote qui les porte un système hétérocyclique choisi parmi pyrrolidine, pipéridine, azépine ou morpholine, le troisième groupement parmi R_{A}, R_{B} et R_{C} étant alors soit un atome d'hydrogène, soit un groupement alkyle, leur N oxyde, leurs isomères ainsi que leurs sels d'addition à un acide ou une base pharmaceutiquement acceptable,
* le composé qui est le N{4-[N-(4,6-diméthylpyridin-2-yl)amino]1,4-dioxobutyl}glycinate d'éthyle, ainsi que son N oxyde, ses sels d'addition à un acide ou une base pharmaceutiquement acceptable,
* le composé qui est le {[N(4,6-diméthylpyridin-2-yl)2-leucinamido]carbonyle}triméthylamino dihydruro bore, ainsi que son N oxyde, ses sels d'addition à un acide ou une base pharmaceutiquement acceptable,
* le composé qui est le N(4,6-diméthylpyridin-2-yl)glycylglycinamide ainsi que son N oxyde, ses sels d'addition à un acide ou une base pharmaceutiquement acceptable,
* le composé qui est le N(4,6-diméthylpyridin-2-yl)glycylglycylglycinamide ainsi que son N oxyde, ses sels d'addition à un acide ou une base pharmaceutiquement acceptable,
* le composé qui est le {[N(4,6-diméthylpyridin-2-yl)2-methioninamido]carbonyle}triméthylamino dihydruro bore, son N oxyde, ses isomères, ainsi que ses sels d'addition à un acide ou une base pharmaceutiquement acceptable,
* le composé qui le{[N(4,6-diméthylpyridin-2-yl)glycinamide son N oxyde, ainsi que ses sels d'addition à un acide ou une base pharmaceutiquement acceptable.

La présente invention a également pour objet le procédé de préparation des composés de formule (I) caractérisé en ce que l'on utilise comme matière première le dérivé de formule (II) : 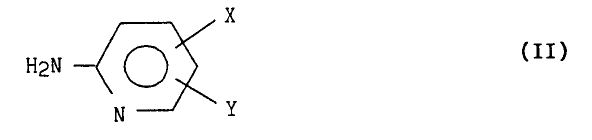
où X et Y ont la même définition que précédemment,
que l'on condense :
* ou bien avec le dérivé de formule (III) : 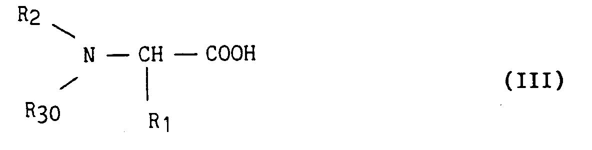
   ou R₁, R₂ ont la même définition que dans la formule (I),
   et R₃₀ représente un groupement t.butoxy carbonyle, benzyloxycarbonyle ou fluorényl méthyloxycarbonyle ou 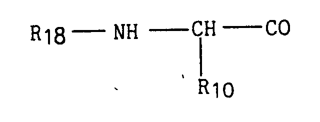
   avec R₁₀ tels que définis précédemment et R₁₈ représentant un groupement t.butoxy carbonyle, benzyloxycarbonyle ou fluorenylméthyloxycarbonyle,
      pour conduire à un dérivé de formule (IV) : 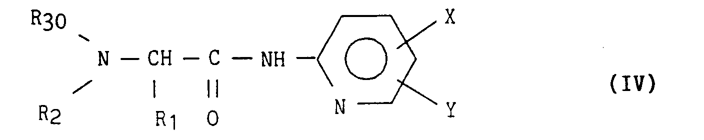
   où R₁, R₂, R₃₀, X et Y ont la même définition que précédemment, cas particulier des dérivés de formule (I) pour lequel A représente un groupement 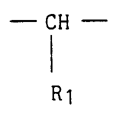
   et R₃ représente un groupement R₃₀ tel que précédemment défini,
      dérivé de formule (IV) que l'on peut ensuite déprotéger pour conduire à un dérivé de formule (V) : 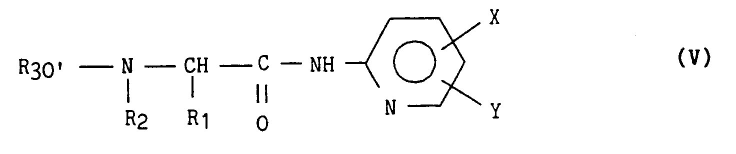
   où R₁, R₂, X et Y ont la même définition que précédemment, et R_{30'} est soit un atome d'hydrogène ou un groupement 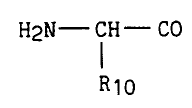 avec R₁₀ tel que défini précédemment
      cas particulier des dérivés de formule (I) pour lequel R₃ représente un groupement R_{30'} tel que défini précédemment
   et A un groupement 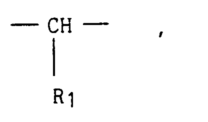
   où R_{30'} et R₁ sont tels que défini précédemment
      dérivé de formule (V), que l'on peut condenser lorsque R_{30'} représente un atome d'hydrogène avec un dérivé de formule (VI) :

      R₉COOH (VI)
   avec R₉ représentant :
      - un groupement phényle,
         un groupement phényle substitué par un groupement alkyle, alkoxyle, hydroxyle, trifluorométhyle ou par un atome d'halogène,
      - un groupement 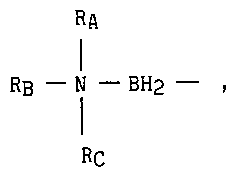 avec R_{A}, R_{B} et R_{C} tels que défini précédemment
      - un groupement 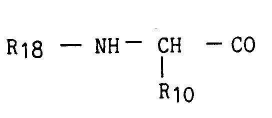
   avec R₁₈ représentant t.butoxycarbonyle, benzyloxycarbonyle, fluorénylméthyloxycarbonyle, glycyl substitué par t. butoxycarbonyle fluorénylméthyloxycarbonyle et R₁₀ conservant la même signification que précédemment,
      pour conduire à un dérivé de formule (VII) 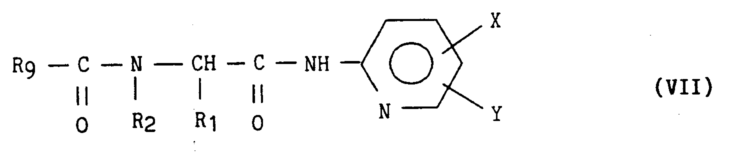
   dans laquelle R, R₂, R₉, X et Y ont la même définition que précédemment, cas particulier des dérivés de formule (I) pour lesquels A représente un groupement 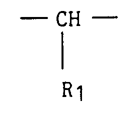 et R₃ un groupement -CO-R₉ avec R₁ et R₉ tels que définis précédemment,
      qui, lorsque R₉ représente un groupement, 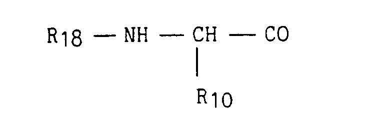
   avec R₁₀ et R₁₈ ayant la même définition que précédemment.
      peut-être si on le désire, soumis à déprotection pour conduire à un dérivé de formule (XVII) 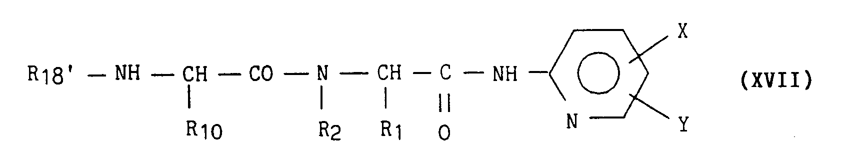
   où R_{18'} représente un atome d'hydrogène ou un radical glycyle et R₁, R₂, R₁₀, X et Y ont la même définition que précédemment,
      cas particulier des dérivés de formule (I) pour lesquels A représente un groupement 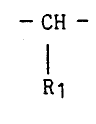 et R₃ un groupement 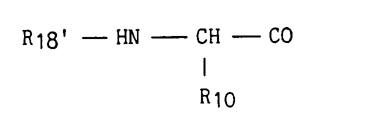
   avec R₁, R_{18'} et R₁₀ ayant la même définition que précédemment
* ou bien avec un dérivé de formule (VIII) : 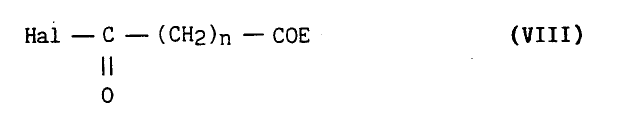
   avec n ayant la même définition que précédemment Hal représentant un atome d'halogène et E représentant un groupement alkoxy, pour obtenir un dérivé de formule (IX) : 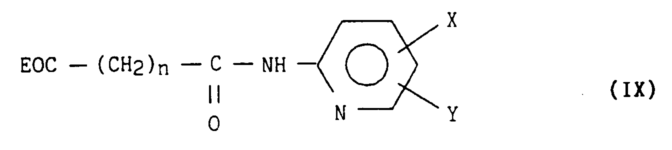
   avec X, Y, n et E ayant la même définition que précédemment qui, traité par un agent alcalin, donne un dérivé de formule (X) : 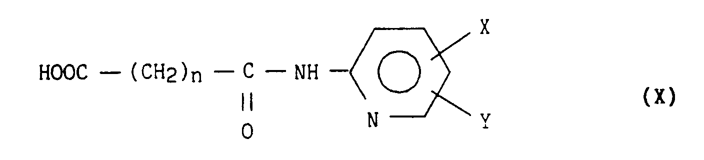
   avec X, Y et n ayant la même définition que précédemment, que l'on traite :
      - soit par un dérivé de formule (XI) : 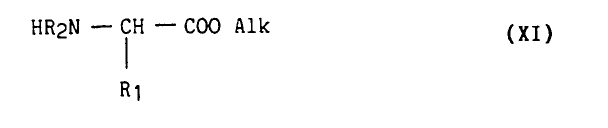
   avec R₁ et R₂ ayant la même définition que précédemment et Alk représente un groupement alkyle,
      pour conduire à un dérivé de formule (XII) : 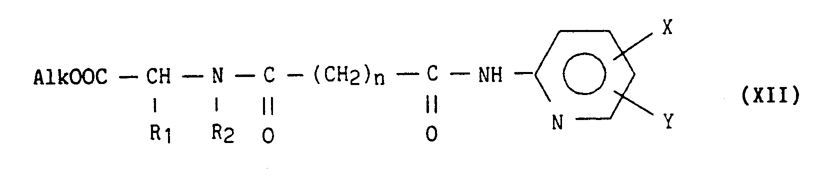 cas particulier de dérivé de formule (I) pour lequel A représente un un groupement (CH₂)ₙCO et R₃ un groupement 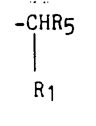 avec R₅ alkoxycarbonyle
   et n, R₁ et R₂ ayant la même définition que précédemment,
      dérivé de formule (XII) qui par déprotection conduit à un dérivé de formule (XIII) : 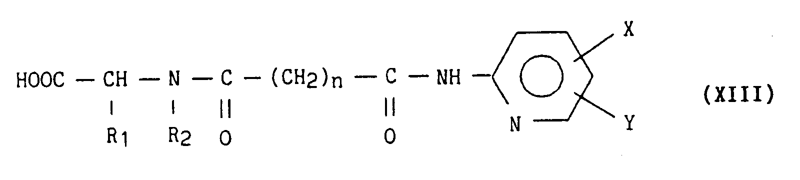 cas particulier des dérivés de formule (I) pour lesquels A représente un un groupement (CH₂)ₙCO et R₃ un groupement 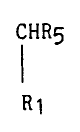 avec R₅ représentant ici un groupement carboxyle, n R₁ et R₂ ayant la même définition que précédémment,
      dérivé de formule (XIII) que l'on peut traiter par un dérivé de formule (XIV) : 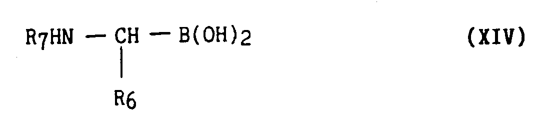
   ou R₆ et R₇ ont la même définition que dans la formule (I),
      éventuellement protégé pour conduire après éventuelle déprotection à un dérivé de formule (XV) : 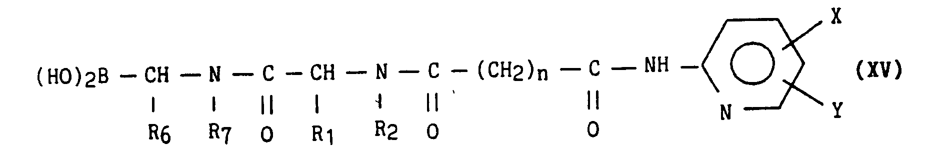 cas particulier des dérivés de formule (I) pour lesquels A représente un groupement -(CH₂)ₙCO- et R₃ un groupement 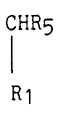 avec R₅ représentant un groupement 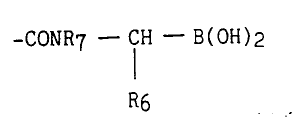
   avec n, R₅, R₁, R₆, R₇ ayant la même définition que dans la formule (I),
      - soit directement par un dérivé de formule (XIV) éventuellement protégé pour conduire après éventuelle déprotection à un dérivé de formule (XVI) : 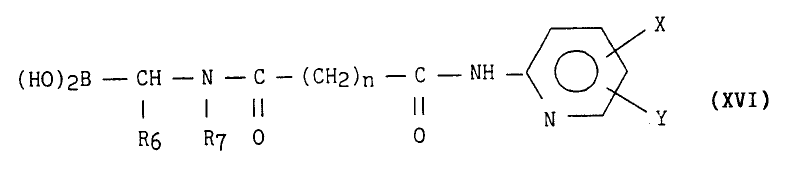
      cas particulier de dérivé de formule (I) pour lequel A représente un
      un groupement 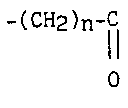 et R₃ un groupement 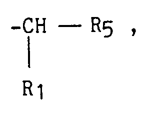 R₅ représentant ici un groupement B(OH)₂ et R₆ ayant la définition préalablement donnée pour R₁, R₇ ayant la définition préalablement donnée pour R₂,
      dérivés de formule (IV), (V), (VII), (XII), (XIII), (XV) et (XVII) que l'on peut, si on le désire, transformer en N oxyde de la pyridine par action d'eau oxygénée,
      dérivés de formule (IV), (V), (VII), (XII), (XIII), (XV), (XVI) et (XVII) et leurs N oxydes dont on sépare éventuellement les isomères et que l'on salifie si on le désire par un acide ou une base pharmaceutiquement acceptable.

Les composés de formule (I) possèdent d'intéressantes propriétés pharmacologiques.

L'étude de ces propriétés a en effet montré que les dérivés de formule (I) n'étaient pas toxiques, doués d'activité antiinflammatoire, hypolipémiante et diurétique.

Ce spectre d'activité rend donc les composés de la présente invention intéressants dans un certain nombre d'indications telles que rhumatismes inflammatoires, polyarthrite rhumatoïde, spondylarthrite ankylosante, arthroses, rhumatismes articulaires, lombalgies, hyperlipédimies, hypertriglycéridémies et hypercholestérolémies, athérosclérose. En outre les composés de l'invention sont actifs également par voie topique, ce qui les rend intéressants dans un certain nombre d'indications cutanées telles que le psoriasis. Enfin de par leur activité diurétique, les composés de l'invention sont utilisables dans les maladies inflammatoires rénales, néphrites, glomérulonéphrites, pyélonéphrites etc...

La présente invention a également pour objet les compositions pharmaceutiques contenant un composé de formule (I), ou un de ses sels d'addition à un acide ou une base pharmaceutiquement acceptable, seul ou en combinaison avec un ou plusieurs excipients pharmacologiquement acceptables.

Parmi les compositions pharmaceutiques selon l'invention, on pourra citer plus particulièrement, à titre d'exemples et de façon non limitative, celles qui conviennent à l'administration orale, parentérale, nasale, rectale, perlinguale, oculaire, cutanée, percutanée, dermique, transdermique ou pulmonaire et notamment les préparations injectables, les aérosols, les gouttes oculaires ou nasales, les comprimés simples, pelliculés ou dragéifiés, les gélules, les capsules, les crèmes, pommades, et gels dermiques.

La posologie utile varie selon l'âge et le poids du patient, la voie d'administration, la nature de l'affection et des traitements éventuellement associés et s'échelonne entre 1 mg et 5 grammes par 24 heures.

Les exemples qui suivent illustrent l'invention et ne la limitent en aucune façon.

La matière première est décrite dans la littérature ou est facilement accessible à l'homme de l'art.

Les spectres infrarouge sont réalisés en pastille de bromure de potassium renfermant environ 1% du produit à analyser.

### STADE A :

A une solution agitée de l'α-aminoacide choisi protégé par un groupement benzyloxycarbonyle ou tert-butoxycarbonyle (10 mmoles) et de carbonyldiimidazole (10 mmoles) dans le tétrahydrofuranne anhydre à température ambiante, on ajoute 10 mmoles de 6-amino-2,4-lutidine. L'agitation à température ambiante est maintenue 10 heures. Le solvant est évaporé sous vide et le résidu purifié par chromatographie sur gel de silice en utilisant l'éther comme éluant.

On obtient ainsi les exemples 1 à 10.

Il peut être intéressant de remplacer le carbonyldiimidazole par une phosphine choisie parmi triphénylphosphine ou tri-n-butylphosphine ou triéthylphosphine dans un solvant chloré tel que BrCCl₃.

### EXEMPLE 1 :

### N-2-BENZYLOXYCARBONYL-N-(4,6-DIMETHYLPYRIDIN-2-YL)GLYCINAMIDE (A = CH₂ : R₂= H)

Rendement : 77%
Point de fusion (éther) : 103°C
Caractéristiques spectrales :
   Infrarouge : 3220, 1710 et 1670 cm⁻¹

### EXEMPLE 2 :

### N-2-TERT.BUTOXYCARBONYL-N-(4,6-DIMETHYLPYRIDIN-2-YL)GLYCINAMIDE (A = CH₂ : R₂= H)

Rendement : 65%
Point de fusion (éther) : 120°C
Caractéristiques spectrales :
   Infrarouge : 3300, 1715 et 1660 cm⁻¹

### EXEMPLE 3 :

### N-2-BENZYLOXYCARBONYL-N-(4,6-DIMETHYLPYRIDIN-2-YL)ALANINAMIDE

Rendement : 70%
Point de fusion (éther) : 121°C
Caractéristiques spectrales :
   Infrarouge : 3200, 1715 et 1665 cm⁻¹

### EXEMPLE 4 :

### N-2-BENZYLOXYCARBONYL-N-(4,6-DIMETHYLPYRIDIN-2-YL)PHENYLALANINAMIDE

Rendement : 60%
Point de fusion (éther) : 139°C
Caractéristiques spectrales :
   Infrarouge : 3260, 1705 et 1660 cm⁻¹

### EXEMPLE 5 :

### N-2-BENZYLOXYCARBONYL-N-(4,6-DIMETHYLPYRIDIN-2-YL)VALINAMIDE

Rendement : 73%
Point de fusion (éther) : 134°C
Caractéristiques spectrales :
   Infrarouge : 3260, 1710 et 1660 cm⁻¹

### EXEMPLE 6 :

### N-2-BENZYLOXYCARBONYL-N-(4,6-DIMETHYLPYRIDIN-2-YL)LEUCINAMIDE

Rendement : 68%
Caractéristiques spectrales :
   Infrarouge : 1720 et 1670 cm⁻¹

### EXEMPLE 7 :

### N-2-BENZYLOXYCARBONYL-N-(4,6-DIMETHYLPYRIDIN-2-YL)ISOLEUCINAMIDE

Rendement : 84%
Point de fusion (éther) : 67°C
Caractéristiques spectrales :
   Infrarouge : 3260, 1710 et 1665 cm⁻¹

### EXEMPLE 8 :

### N-2-BENZYLOXYCARBONYL-N-(4,6-DIMETHYLPYRIDIN-2-YL)METHIONINAMIDE

Rendement : 87%
Point de fusion (éther) : 70°C
Caractéristiques spectrales :
   Infrarouge : 3320, 3200, 1730 et 1660 cm⁻¹

### EXEMPLE 9 :

### N-2-TERT.BUTOXYCARBONYL-N-(4,6-DIMETHYLPYRIDIN-2-YL)METHIONINAMIDE

Rendement : 60%
Point de fusion (éther) : 84°C
Caractéristiques spectrales :
   Infrarouge : 3330, 1720 et 1660 cm⁻¹

### EXEMPLE 10 :

### N-2-BENZYLOXYCARBONYL-N-(4,6-DIMETHYLPYRIDIN-2-YL)PROLINAMIDE

Rendement : 73%
Point de fusion (éther) : 149°C
Caractéristiques spectrales :
   Infrarouge : 3240, 1705 et 1681 cm⁻¹

### STADE B :

Les composés N-2-benzyloxycarbonylcarboxamides obtenus aux exemples 1, 3, 4, 6, 7 (100 mmol) sont dissous dans du méthanol et on ajoute 0,1 mmol decatalyseur palladié sur charbon (5%). Le mélange est agité à température ambiante sous atmosphère d'hydrogène jusqu'à cessation d'absorption du gaz. Le catalyseur est éliminé par filtration et le filtrat concentré sous vide pour donner les exemples 11 à 15.

### EXEMPLE 11 :

### N-(4,6-DIMETHYLPYRIDIN-2-YL)GLYCINAMIDE

Rendement : 75%
Point de fusion : 105°C
Caractéristiques spectrales :
   Infrarouge : 3060, 3140 et 1690 cm⁻¹

### EXEMPLE 12 :

### N-(4,6-DIMETHYLPYRIDIN-2-YL)ALANINAMIDE

Rendement : 56%
Caractéristiques spectrales :
   Infrarouge : 3300, 1670 cm⁻¹

### EXEMPLE 13 :

### N-(4,6-DIMETHYLPYRIDIN-2-YL)PHENYLALANINAMIDE

Rendement : 60%
Point de fusion : 97°C (éther diéthylique)
Caractéristiques spectrales :
   Infrarouge : 3240, 1660 cm⁻¹

### EXEMPLE 14 :

### N-(4,6-DIMETHYLPYRIDIN-2-YL)LEUCINAMIDE

Rendement : 55%
Point de fusion : 78°C (éther diéthylique)
Caractéristiques spectrales :
   Infrarouge : 3360, 1665 cm⁻¹

### EXEMPLE 15 :

### N-(4,6-DIMETHYLPYRIDIN-2-YL)ISOLEUCINAMIDE

Rendement : 58%
Caractéristiques spectrales :
   Infrarouge : 3300, 1655 cm⁻¹

### EXEMPLE 16 :

### N-(4,6-DIMETHYLPYRIDIN-2-YL)METHIONINAMIDE 10 mmol du produit obtenu dans l'exemple 9 sont agités une heure dans l'acide trifluoroacétique à température ambiante. L'excès d'acide trifluoroacétique est évaporé sous vide pour donner le trifluoroacétate déplacé dans le chlorure de méthylène par la triéthylamine.

Rendement : 60%
Caractéristiques spectrales :
   Infrarouge : 3240, 1650 cm⁻¹

### EXEMPLE 17 :

### 2-(3-FLUORO)BENZOYL-N'-(4,6-DIMETHYLPYRIDIN-2-YL)GLYCINAMIDE

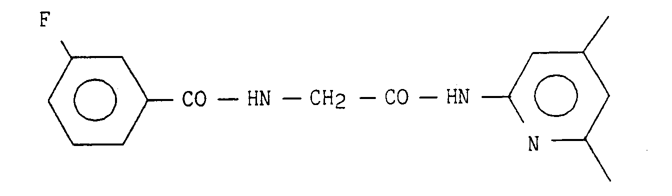

Porter au reflux de l'acide 3-fluoro benzoïque dans du chlorure de thionyle en présence de diméthyl formamide pendant une heure. Eliminer le chlorure de thionyle sous vide pour permettre l'obtention du chlorure de 3-fluoro benzoyle. Dissoudre ce dérivé dans l'éther et ajouter la solution ainsi obtenue à une solution de N-(4,6-diméthylpyridin-2-yl) glycinamide, obtenue dans l'exemple 11, dans l'éther, en présence de triéthylamine. Agiter trois heures à température ambiante. Eliminer par filtration le sel de triéthylamine et le solvant par évaporation sous vide. Purifier le résidu par chromatographie sur silice en utilisant l'éther comme éluant.
Rendement : 53%
Point de fusion : 161°C
Caractéristiques spectrales :
   Infrarouge : 3260, 1680 cm⁻¹

### EXEMPLE 18 :

### {[N(4,6-DIMETHYLPYRIDIN-2-YL)2-GLYCINAMIDO]CARBONYL}TRIMETHYLAMINO DIHYDRURO BORE

En utilisant le couple P(Ph)₃/CCl₄ en présence de carboxytriméthylamino dihydruro bore sur le composé de l'exemple 11, on obtient selon SPIELVOGEL et Col (J. Am. Chem. Soc. 1976, 100, 5702-5703) le produit du titre de formule : 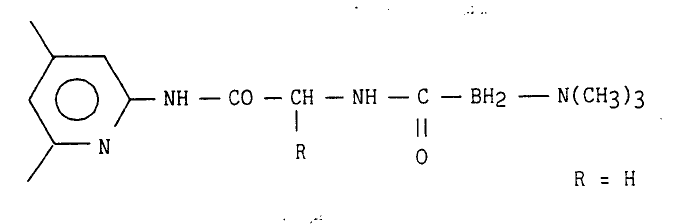
Rendement : 48%
Point de fusion : 106°C

En procédant à l'identique avec les exemples 13, 14 et 16, on obtient :

### EXEMPLE 19 :

### {[N(4,6-DIMETHYLPYRIDIN-2-YL)2-PHENYLALANINAMIDO]CARBONYL}TRIMETHYLAMINO DIHYDRURO BORE (R = benzyle)

Rendement : 51%
Point de fusion : 82°C

### EXEMPLE 20 :

### {[N(4,6-DIMETHYLPYRIDIN-2-YL)2-LEUCINAMIDO]CARBONYL}TRIMETHYLAMINO DIHYDRURO BORE (R = CH₂-CH(CH₃)₂)

Rendement : 45%, huile

### EXEMPLE 21 :

### {[N(4,6-DIMETHYLPYRIDIN-2-YL)2-METHIONINAMIDO]CARBONYL}TRIMETHYLAMINO DIHYDRURO BORE (R = -CH₂CH₂-S-CH₃)

Rendement : 46%
Point de fusion : 120°C

### EXEMPLE 22 :

### {[N(4,6-DIMETHYLPYRIDIN-2-YL)2-GLYCINAMIDO]CARBONYL}N METHYL PIPERIDINO DIHYDRURO BORE

En procédant comme dans l'exemple 18, mais en remplaçant le carboxy triméthylamino dihydruro bore par le carboxy N méthyl pipéridino dihydruro bore, on obtient le produit du titre.
Rendement : 58%
Point de fusion : 140°C

### EXEMPLE 23 :

### N{4-{[(4,6-DIMETHYLPYRIDIN-2-YL)AMINO]1,4-DIOXO}BUTYL}GLYCINATE D'ETHYLE

### STADE A :

Faire réagir dans le chlorure de méthylène en présence de triéthylamine 100 mmol de 6-amino 2,4-lutidine en présence de 100 mmole d'ester éthylique de l'acide 4-chloro 4-oxo butanoïque. Filtrer le sel formé, évaporer le milieu réactionnel et purifier sur gel de silice l'ester éthylique de l'acide 4-(4,6-diméthylpyridin-2 yl)amino 4-oxo butanoïque.

### STADE B :

10 mmole du produit obtenu au stade A sont agités une heure dans la soude 0,1N. Par addition lente d'un acide faible, l'acide 4-(4,6-diméthyl pyridin -2 yl) amino 4-oxobutanoïque précipite.

### STADE C :

Condenser l'acide 4-(4,6-diméthylpyridin-2-yl)amino 4-oxobutanoïque avec le glycinate d'éthyle en présence de triphénylphospine dans un mélange de trichlorobromométhane et tétrahydrofurane pour obtenir le produit du titre.

### EXEMPLE 24 :

### BENZYLOXYCARBONYL N-(4,6-DIMETHYL PYRIDIN-2-YL)GLYCYLGLYCINAMIDE

Dissoudre 2g de benzyloxycarbonyl glycine dans 30 ml de tétrahydrofuranne sec. Ajouter du carbonyle diimidazole (1.62 g, 10 mmol).Laisser 1 h sous agitation à température ambiante avant d'ajouter le produit de l'exemple 11 (1.80 10 mmol). Après 6 h sous agitation à température ambiante, filtrer le produit du titre et le laver au THF.

### EXEMPLE 25 :

### N-(4,6-DIMETHYL PYRIDIN-2-YL)GLYCYLGLYCINAMIDE

Dissoudre 1g (2.6 mmol) du produit de l'exemple 24 dans 60 ml de méthanol. Ajouter du charbon palladié et agiter sous atmosphère d'hydrogène. Filtrer, évaporer le méthanol et recueillir le produit du titre.
Point de fusion : décomposition au dessus de 180°C
Caractéristiques spectrales :
   RMN ¹H 3.99 ppm singulet 2H (CH₂ NH)

### EXEMPLE 26 :

### N-(4,6-DIMETHYL PYRIDIN-2-YL)GLYCYLGLYCYLGLYCINAMIDE

A une solution agitée de benzyloxycarbonyl glycylglycine (10 mmoles) et de carbonyl diimidazole (10 mmoles) dans le tétrahydrofurane anhydre à température ambiante, on ajoute 10 mmoles de N-(4,6-diméthyl pyridin-2-yl)glycinamide obtenu à l'exemple 11. L'agitation à température ambiante est maintenue 10 heures. Le solvant est évaporé sous vide et le résidu purifié par chromatographie sur gel de silice en utilisant l'éther comme éluant.

### EXEMPLE 27 :

### N OXYDE DE LA N-4,6-DIMETHYL PYRIDIN-2-YL)GLYCINAMIDE

Ajouter sous agitation 1 g de composé obtenu dans l'exemple 11 dans une solution de 10 ml d'acide acétique glacial et 0,7 ml d'eau oxygénée (35%). Chauffer le milieu réactionnel à 70°C pendant 7 heures, puis concentrer sous pression réduite à basse température. Filtrer. Laver à l'eau glacée, sécher, purifier par chromatographie sur gel de silice. Recristalliser.

### ETUDE PHARMACOLOGIQUE DES DERIVES DE L'INVENTION

### EXEMPLE 28 :

### ETUDE DE LA TOXICITE AIGUE

La toxicité aiguë a été appréciée après administration orale à des lots de 8 souris ( 26 ± 2 grammes) d'une dose de 650 mg.kg⁻¹. Les animaux ont été observés à intervalles réguliers au cours de la première journée et quotidiennement pendant les 2 semaines suivant le traitement.
Il apparaît que les composés de l'invention sont totalement atoxiques. Aucun décès n'est observé après administration d'une dose de 650 mg.kg⁻¹. On ne constate pas de troubles après administration de cette dose.

### EXEMPLE 29 :

### ETUDE DE L'ACTIVITE ANTIINFLAMMATOIRE

La méthode retenue est celle de l'oedème plantaire à la carraghénine. Le protocole utilisé est le suivant : on administre à des souris male CF1 de poids moyen 25 g, les composés de l'invention à une dose de 100 mg.kg-1 en suspension à 0,05% dans le mélange de Tween 80-Eau par voie intrapéritonéale. Cette administration est réalisée dans la plante du pied droit durant 3 heures puis à nouveau 30 minutes avant l'injection de 0,2 ml d'une solution saline à 9% de carraghénine à 1%. Cette solution saline est également injectée dans la plante du pied gauche qui sert de témoin. Trois heures plus tard les deux pieds sont coupés au niveau de l'articulation tibio tarsale selon la méthode de Winter modifiée (J. Pharmcol. Exp. Ther. 1970, 175, 435-442 et Clin. Chim. Acta 10, 229-237).

Le pied gauche voit son poids augmenter de 78 ± 3 mg.
Le poids du pied droit n'augmente pas grâce à l'efficacité totale des produits de l'invention.

### EXEMPLE 30 :

### ACTIVITE HYPOLIPIDEMIANTE

On traite des souris male CF1 de poids moyen 28 g par les produits de l'invention (en suspension à 1% dans de la carboxyméthyl cellulose) à raison de 20 mg/kg par jour par voie intrapéritonéale pendant 16 jours. Aux jours 9 et 16, on réalise en prélèvement sanguin sur les animaux. Le plasma est obtenu par centrifugation (3 min × 3000 g). Le cholestérol total est déterminé par la réaction de Liebermann-Buchard (Clin. Chim. Acta, 1964, 10, 229-237) et les triglycérides sont déterminés à l'aide d'un kit commercial (Bio Dynamics / bmc triglycéride kit). Les produits de l'invention administrés par voie intrapéritonéle à raison de 20 mg.kg-1 par jour permettent un abaissement d'environ 70% du cholestérol et des triglycérides plasmatiques.

### EXEMPLE 31 :

### ETUDE DE L'ACTIVITE DIURETIQUE

On utilise des groupes de 3 rats à jeun. Chaque groupe reçoit 25 ml/kg p.o. d'eau distillé administrée avec les produits de l'invention (30mg/kg). Le volume urinaire est mesuré pendant les 6 heures suivant l'administration. Le pouvoir diurétique des produits de l'invention est comparable à celui du furosémide pris comme référence.

### EXEMPLE 32 :

### COMPOSITIONS PHARMACEUTIQUES

. Comprimés pour le traitement des maladies inflammatoires
Composés dosés à 10 mg de N-(4,6-diméthylpyridin-2-yl)glycyl glycinamide.
Formule de préparation pour 1000 comprimés

| | |
|---|---|
| N-(4,6-diméthyl pyridin-2-yl)glycyl glycinamide | 10 g |
| Amidon de blé | 35 g |
| Amidon de maïs | 65 g |
| Lactose | 65 g |
| Stéarate de Magnésium | 2 g |
| Silice | 1 g |
| Hydroxypropylcellulose | 2 g |

. Comprimés destinés au traitement des hypercholestérolémies et hypertriglycéridémies dosés à 15 mg de {[4,6-diméthylpyridin-2-yl) 2-(phénylalaninamido) carbonyl]}triméthylamino dihydruro bore.
Formule de préparation pour 1000 comprimés.

| | |
|---|---|
| {[4,6-diméthylpyridin-2-yl) 2-(phénylalaninamido) carbonyl]}triméthylamino dihydruro bore | 15 g |
| Amidon de blé | 35 g |
| Amidon de maïs | 65 g |
| Lactose | 65 g |
| Stéarate de Magnésium | 2 g |
| Silice | 1 g |
| Hydroxypropylcellulose | 2 g. |

. Pommade destinée au traitement du psoriasis dosée à 1 % de N(4,6-diméthylpyridin-2-yl)glycyl glycinamide.
Formule de préparation pour 1000 kilogrammes.
N(4,6-diméthylpyridin-2-yl)glycylglycynamide 1kg.
Excipients QSP 1000 kg
(Alcools cetylique, stéarylique, isopropylique, lanoline, monostéréate de polyéthylène glycolle, eau distillée de laurier cerise).

## Revendications

1. Composés de formule générale (I) : 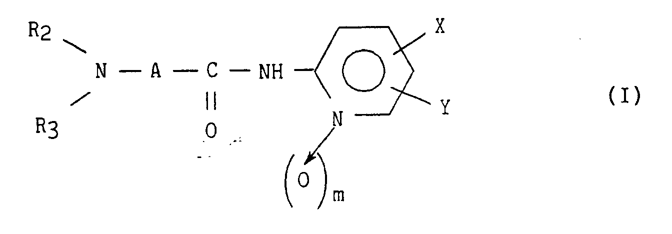 dans laquelle :
m représente 0 ou 1,
X et Y identiques ou différents représentent chacun un groupement alkyle,
A représente :
1) - un groupement 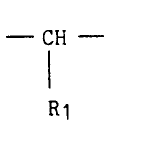
et R₁ représente :
- un atome d'hydrogène,
- un groupement alkyle,
- un groupement alkyle substitué par un groupement aminé,
- un groupement alkylthioalkyle,
- un groupement alkyle substitué par un groupement aminé lui-même substitué sur le groupe amino par un groupement choisi parmi tert-butoxycarbonyle, benzyloxycarbonyle, fluorénylméthyloxy carbonyle,
- un groupement phényle ou phényl alkyle,
- un groupement phényle ou phénylalkyle substitué sur le noyau aromatique par un groupement alkyle, hydroxyle, alkoxyle, trifluorométhyle ou par un atome d'halogène,
et dans ce cas - où A représente un groupement -CH(R₁)- :
R₂ représente un atome d'hydrogène ou R₂ forme avec R₁ et le chaînon CH-N- qui les porte, un système carboné mono ou bicyclique renfermant le chaînon -CH-N-, chaque cycle comprenant de 5 ou 6 sommets et pouvant être saturé ou non,
et alors,
R₃ représente :
- un atome d'hydrogène,
- un groupement t.butoxycarbonyle, benzyloxycarbonyle, fluorénylméthyloxycarbonyle,
- un groupement benzoyle,
- un groupement benzoyle substitué par un groupement alkyle, alkoxy, hydroxyle, trifluorométhyle ou par un atome d'halogène,
- un groupement 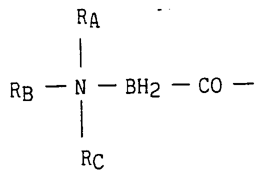 avec R_{A}, R_{B}, R_{C} identiques ou différents représentant un atome d'hydrogène ou un groupement alkyle, ou encore deux groupements parmi R_{A}, R_{B}, R_{C} forment avec l'atome d'azote qui les porte un système hétérocyclique choisi parmi pyrrolidine, pipéridine, azépine ou morpholine, le troisième groupement parmi R_{A}, R_{B} et R_{C} étant alors soit un atome d'hydrogène, soit un groupement alkyle,
- un groupement 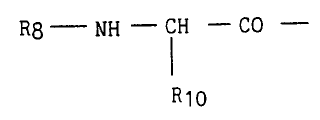
avec R₈ représentant hydrogène, t.butoxycarbonyle, benzyloxycarbonyle, fluorényl méthyloxycarbonyle, glycyle, glycyle substitué par un groupement t.butoxycarbonyle, benzyloxycarbonyle, fluorénylméthyloxycarbonyle et R₁₀ représentant hydrogène ; alkyle ; alkyle substitué par un groupement amino ; alkyle substitué par un groupement amino lui-même substitué par un groupement t.butoxycarbonyle, benzyloxycarbonyle, fluorénylméthyloxycarbonyle ; un groupement phényle ou phénylalkyle, un groupement phényle ou phénylalkyle substitué sur le noyau phényle par un groupement alkyle, hydroxyle, alkoxyle, trifluorométhyle ou par un atome d'halogène,
2) - un groupement 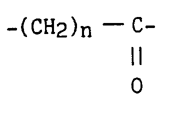 le groupement 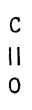 du radical A étant lié au groupement 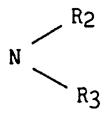 du dérivé de formule (I) avec n entier compris inclusivement entre 1 et 6,
et dans ce cas R₃ représente un groupement 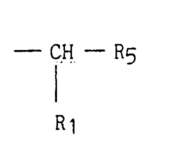
R₁ et R₂ conservant la définition donnée précédemment, et R₅ représentant un groupement B(OH)₂, carboxyle, alkoxycarbonyle,
ou bien 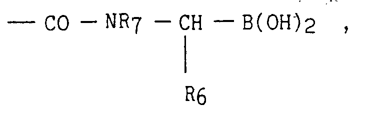
avec R₆ et R₁ identiques ou différents et ayant chacun la définition de R₁ telle que donnée préalablement, R₇ ayant la définition de R₂ donnée précédemment et étant identique à R₂ ou différent,
leurs isomères, épimères, énantiomères, diastéréoisomères ainsi que leurs sels d'addition à un acide ou une base pharmaceutiquement acceptable,
étant entendu que par alkyle ou par alkoxy on entend des groupements linéaires ou ramifiés comprenant de 1 à 6 atomes de carbone.

2. Composés selon la revendication 1 ayant la formule générale (I) : 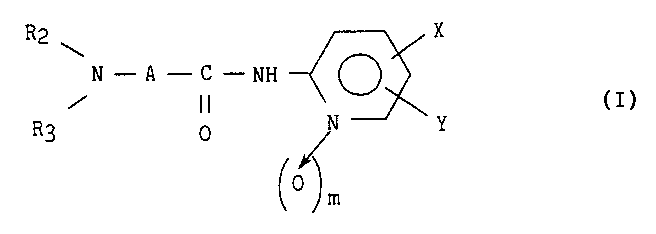 dans laquelle :
m représente 0 ou 1,
X et Y identiques ou différent représentent chacun un groupement alkyle,
et A représente un groupement 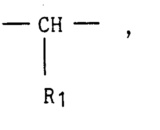
et R₁ représente :
- un atome d'hydrogène,
- un groupement alkyle,
- un groupement alkyle substitué par un groupement aminé,
- un groupement alkylthioalkyle,
- un groupement alkyle substitué par un groupement aminé lui-même substitué sur le groupe amino par un groupement choisi parmi tert-butoxy carbonyle, benzyloxycarbonyle, fluorénylméthyloxycarbonyle,
- un groupement phényle ou phénylalkyle,
- un groupement phényle ou phénylalkyle sur le noyau aromatique substitué par un groupement alkyle, hydroxyle, alkoxyle, trifluorométhyle ou par un atome d'halogène,
R₂ représente un atome d'hydrogène ou R₂ forme avec R₁ et le chaînon CH-N- qui les porte, un système carboné mono ou bicyclique renfermant le chaînon -CH-N-, chaque cycle comprenant de 5 ou 6 sommets et pouvant être saturé ou non,
et,
R₃ représente :
- un atome d'hydrogène,
- un groupement t.butoxycarbonyle, benzyloxycarbonyle, fluorénylméthyloxycarbonyle,
- un groupement benzoyle,
- un groupement benzoyle substitué par un groupement alkyle, alkoxy, hydroxyle, trifluorométhyle ou par un atome d'halogène,
- un groupement 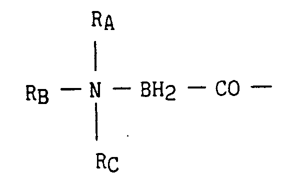 avec R_{A}, R_{B}, R_{C} identiques ou différents représentant un atome d'hydrogène ou un groupement alkyle, ou encore deux groupements parmi R_{A}, R_{B}, R_{C} forment avec l'atome d'azote qui les porte un système hétérocyclique choisi parmi pyrrolidine, pipéridine, azépine ou morpholine, le troisième groupement parmi R_{A}, R_{B} et R_{C} étant alors soit un atome d'hydrogène, soit un groupement alkyle,
leurs isomères, épimères, énantiomères, diastéréoisomères ainsi que leurs sels d'addition à un acide ou une base pharmaceutiquement acceptable,
étant entendu que par alkyle ou par alkoxyle on entend des groupements linéaires ou ramifiés comprenant de 1 à 6 atomes de carbone.

3. Composés de formule générale (I) selon la revendication 1 : 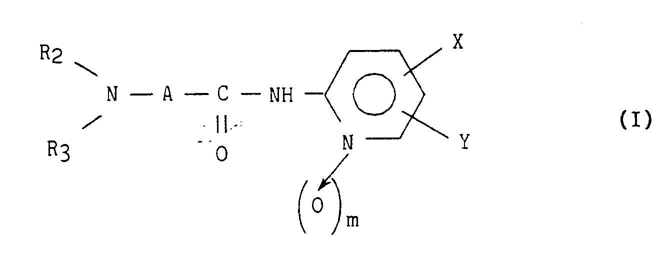 dans laquelle :
m représente 0 ou 1,
X et Y identiques ou différents représentent chacun un groupement alkyle
et A représente un groupement 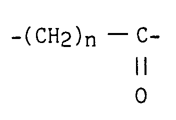 le groupement 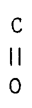 du radical A étant lié au groupement 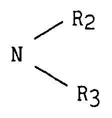 du dérivé de formule (I) avec n entier compris inclusivement entre 1 et 6,
R₃ représente un groupement 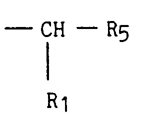
et R₁ représente :
- un atome d'hydrogène,
- un groupement alkyle,
- un groupement alkyle substitué par un groupement aminé,
- un groupement alkylthioalkyle,
- un groupement alkyle substitué par un groupement aminé lui-même substitué sur le groupe amino par un groupement choisi parmi tert butoxy carbonyle, benzyloxycarbonyle, fluorénylméthyloxycarbonyle,
- un groupement phényle ou phényl alkyle,
- un groupement phényle ou phényl alkyle sur le noyau aromatique substitué par un groupement alkyle, hydroxyle, alkoxy, trifluorométhyle ou par un atome d'halogène,
R₂ représentant un atome d'hydrogène ou R₂ forme avec R₁ et le chaînon CH-NR₃ qui les porte, un système carboné mono ou bicyclique renfermant le chaînon -CH-N(R₃), chaque cycle comprenant de 5 ou 6 sommets et pouvant être saturé ou non,
avec R₅ représentant un groupement B(OH)₂, carboxyle, alkoxy carbonyle,
ou bien 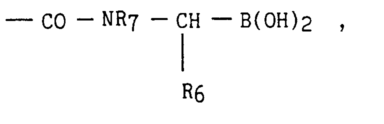
avec R₆ et R₁ identiques ou différents et ayant chacun la définition de R₁ telle que donnée préalablement, R₇ ayant la définition de R₂ donnée précédemment et étant identique à R₂ ou différent,
leurs isomères, épimères, énantiomères, diastéréoisomères ainsi que leurs sels d'addition à un acide ou une base pharmaceutiquement acceptable.

4. Composés de formule générale (I/A) selon la revendication 1 : 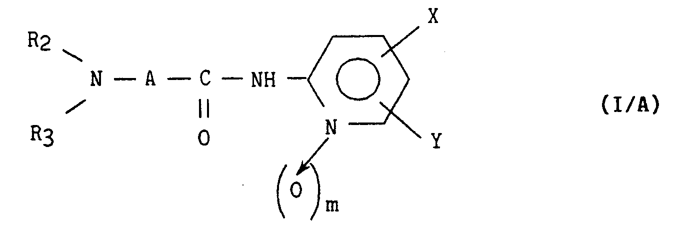
dans laquelle m représente 0 ou 1, X et Y identiques ou différents représentent chacun un groupement alkyle et A représente un groupement 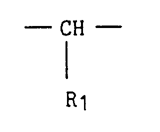
et R₁ représente :
- un atome d'hydrogène,
- un groupement alkyle substitué par un groupement aminé,
- un groupement alkylthioalkyle,
- un groupement alkyle substitué par un groupement aminé lui-même substitué sur le groupe amino par un groupement choisi parmi tert-butoxycarbonyle, benzyloxycarbonyle, fluorénylméthyloxycarbonyle,
- un groupement phényle ou phénylalkyle,
- un groupement phényle ou phénylalkyle sur le noyau aromatique substitué par un groupement alkyle, hydroxyle, alkoxyle, trifluorométhyle ou par un atome d'halogène,
et R₃ représente :
- un groupement 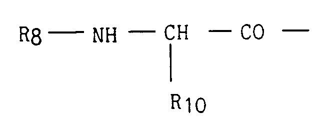
avec R₈ représentant hydrogène, t.butoxycarbonyle, benzyloxycaronyle, fluorénylméthyloxycarbonyle et R₁₀ représentant hydrogène ; alkyle ; alkyle substitué par un groupement amino ; alkyle substitué par un groupement amino lui-même substitué par un groupement t.butoxycarbonyle, benzyloxycarbonyle, fluorénylméthyloxycarbonyle ; un groupement phényle ou phénylalkyle, un groupement phényle ou phénylalkyle substitué sur le noyau phényle par un groupement alkyle, hydroxyle, alkoxyle, trifluorométhyle ou par un atome d'halogène,
leurs isomères, épimères, énantiomères, diastéréoisomères ainsi que leurs sels d'addition à un acide ou une base pharmaceutiquement acceptable.

5. Composés de formule (I/A) selon la revendication 1 : 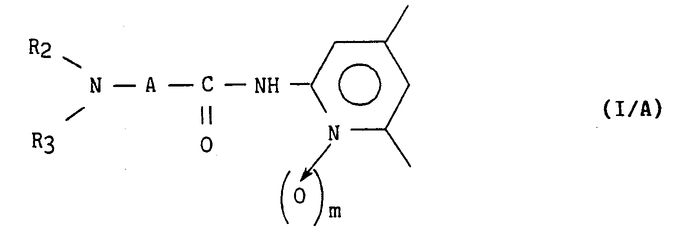
avec m représentant 0 ou 1, A représentant un groupement 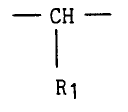 et R₁ choisi parmi hydrogène, alkyle, benzyle, R₂ représente hydrogène, benzyloxy carbonyle, t.butoxy carbonyle et R₃ représente : 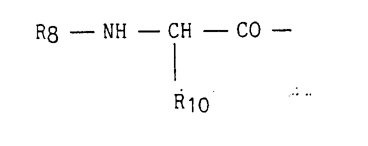
avec R₈ représentant hydrogène, t.butoxycarbonyle, benzyloxycarbonyle, fluorénylméthyloxycarbonyle, glycyle, glycyle substitué par un groupement t.butoxycarbonyle benzyloxycarbonyle, fluorénylméthyloxycarbonyle et R₁₀ représentant hydrogène ; alkyle ; alkyle substitué par un groupement amino ; alkyle substitué par un groupement amino lui-même substitué par un groupement t.butoxycarbonyle, benzyloxycarbonyle, fluorénylméthyloxycarbonyle ; un groupement phényle ou phénylalkyle, un groupement phényle ou phénylalkyle substitué sur le noyau phényle par un groupement alkyle, hydroxyle, alkoxyle, trifluorométhyle ou par un atome d'halogène,
leurs isomères épimères, énantiomères, diastéréoisomères ainsi que leurs sels d'addition à un acide ou une base pharmaceutiquement acceptable.

6. Composés de formule (I/A) selon la revendication 1 : 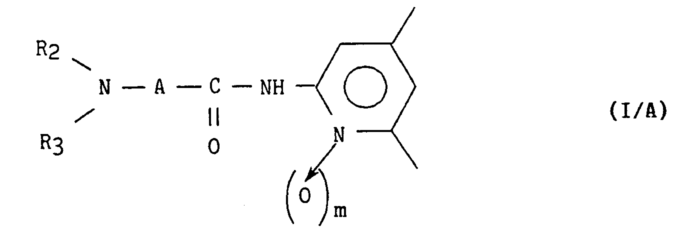
avec m représentant 0 ou 1 et A représentant un groupement 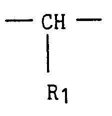 et R₁ choisi parmi hydrogène, alkyle, benzyle ou R₂ représente hydrogène et R₃ un groupement 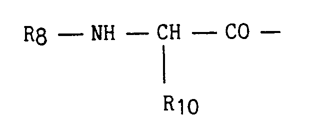
avec R₈ représentant hydrogène, t.butoxycarbonyle, benzyloxycarbonyle, fluorénylméthyloxycarbonyle, glycyle, glycyle substitué par un groupement t.butoxycarbonyle benzyloxycarbonyle, fluorénylméthyloxycarbonyle et R₁₀ représentant hydrogène ; alkyle alkyle substitué par un groupement amino ; alkyle substitué par un groupement amino lui-même substitué par un groupement t.butoxycarbonyle, benzyloxycarbonyle, fluorényl méthyloxycarbonyle ; un groupement phényle ou phénylalkyle, un groupement phényle ou phénylalkyle substitué sur le noyau phényle par un groupement alkyle, hydroxyle, alkoxyle, trifluorométhyle ou par un atome d'halogène, leurs isomères, épimères, énantiomères, diastéréoisomères ainsi que leurs sels d'addition à un acide ou une base pharmaceutiquement acceptable.

7. Composé selon la revendication 1 qui est le N-2-benzyloxycarbonyl N-(4,6-diméthylpyridin-2-yl) prolinamide, son N oxyde, ses isomères, ainsi que ses sels d'addition à un acide ou une base pharmaceutiquement acceptable.

8. Composé selon la revendication 1 qui est le N-2-benzyloxycarbonyl-N-(4,6-diméthylpyridin-2-yl)glycinamide, son N oxyde, ainsi que ses sels d'addition à un acide ou une base pharmaceutiquement acceptable.

9. Composé selon la revendication 1 qui est le N-2-(3-fluoro)benzoyl N-(4,6-diméthyl pyridin-2 yl) glycinamide, son N oxyde, ainsi que ses sels d'addition à un acide ou une base pharmaceutiquement acceptable.

10. Composés de formule I selon la revendication 1 pour lesquels A représente un groupement : 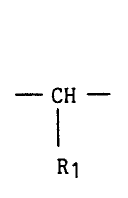 et R₃ un groupement 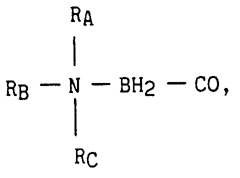 avec R_{A}, R_{B}, R_{C}
identiques ou différents représentant un atome d'hydrogène ou un groupement alkyle, ou encore deux groupements parmi R_{A}, R_{B}, R_{C} forment avec l'atome d'azote qui les porte un système hétérocyclique choisi parmi pyrrolidine, pipéridine, azépine ou morpholine, le troisième groupement par R_{A}, R_{B}, et R_{C} étant alors soit un atome d'hydrogène, soit un groupement alkyle, identiques ou différents de leurs isomères ainsi que leur N oxyde leurs sels d'addition à un acide ou une base pharmaceutiquement acceptable.

11. Composé selon la revendication 1 qui est le N{[1,4-dioxo 4-(4,6-diméthylpyridin-2-yl)amino]butyl}glycinate d'éthyle, ainsi que son N oxyde, ses sels d'addition à un acide ou une base pharmaceutiquement acceptable.

12. Composé selon la revendication 1 qui est le {[N(4,6-diméthylpyridin-2-yl)2-leucinamido]carbonyl}triméthylamino dihydruro bore, ainsi que son N oxyde, ses sels d'addition à un acide ou une base pharmaceutiquement acceptable.

13. Composé selon la revendication 1 qui est le {[N(4,6-diméthylpyridin-2-yl)2-methioninamido]carbonyl}triméthylamino dihydruro bore, son N oxyde, ses isomères, ainsi que ses sels d'addition à un acide ou une base pharmaceutiquement acceptable.

14. Composé selon la revendication 1 qui est le N(4,6-diméthylpyridin-2-yl)glycinamide ainsi que son N oxyde, ses sels d'addition à un acide ou une base pharmaceutiquement acceptable.

15. Composé selon la revendication 1 qui est le N(4,6 diméthyl pyridin-2-yl)glycyl glycinamide ainsi son N oxyde, que ses sels d'addition à un acide ou une base pharmaceutiquement acceptable.

16. Composé selon la revendication 1 qui est le N(4,6 diméthyl pyridin-2-yl)glycylglycylglycinamide ainsi que son N oxyde, ses sels d'addition à un acide ou une base pharmaceutiquement acceptable.

17. Procédé de synthèse des composés de formule (I) selon la revendication 1, pour lesquels :
A représente un groupement 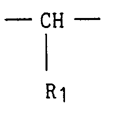 caractérisé en ce que l'on utilise
comme matière première un dérivé de formule (II) : 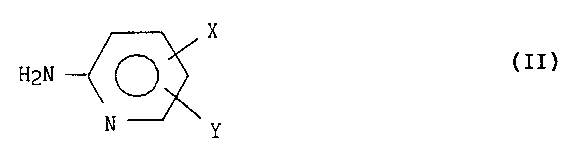
avec X et Y identiques ou différents représentant chacun un groupement alkyle
que l'on condense avec le dérivé de formule (III) : 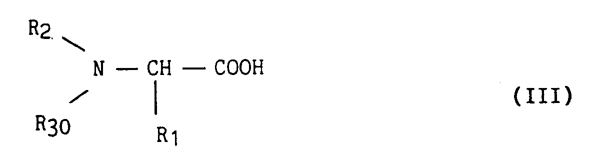
ou R₁, R₂ ont la même définition que dans la formule (I),
et R₃₀ représente un groupement t.butoxy carbonyle, benzyloxycarbonyle ou fluorényl méthyloxycarbonyle, ou 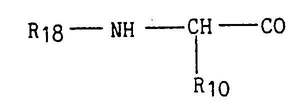
avec R₁₀ tels que définis précédemment et R₁₈ représentant un groupement t.butoxycarbonyle, benzyloxycarbonyle ou fluorenylméthyloxycarbonyle,
pour conduire à un dérivé de formule (IV) : 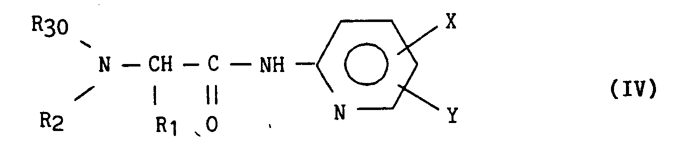 cas particulier des dérivés de formule (I) pour lequel A représente un un groupement 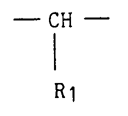 et R₃ représente un groupement R₃₀ tel que précédemment défini,
dérivé de formule (IV) que l'on peut ensuite déprotéger pour conduire à un dérivé de formule (V) : 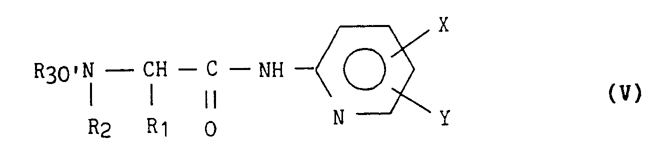
avec R₁, R₂, X et Y tels que défini précédemment, et R_{30'} représente soit un atome d'hydrogène soit un groupement 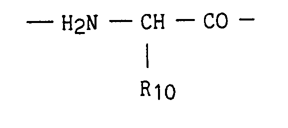
avec R₁₀ tel que défini précédemment
cas particulier des dérivés de formule (I) pour lequel R₃ représente un groupement R_{30'} et A un groupement 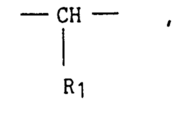
avec R₁ et R_{30'} tels que définis précédemment
dérivé de formule (V) que l'on peut condenser lorsque R_{30'} représente un atome d'hydrogène avec un dérivé de formule (VI) :
R₉COOH (VI)
avec R₉ représentant :
- un groupement phényle,
un groupement phényle substitué par un groupement alkyle, alkoxyle, hydroxyle, trifluorométhyle ou par un atome d'halogène,
- un groupement 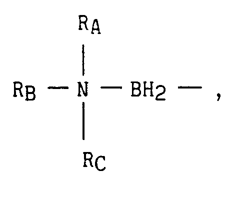 avec R_{A}, R_{B} et R_{C} tels que défini précédemment
- un groupement 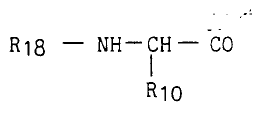
avec R₁₈ représentant t.butoxycarbonyle, benzyloxycarbonyle, fluorénylméthyloxycarbonyle, glycyle, glycyle substitué par un groupement t.butoxcarbonyle, benzyloxycarbonyle, fluorénylméthyloxycarbonyle et R₁₀ conservant la même signification que précédemment, pour conduire à un dérivé de formule (VII) : 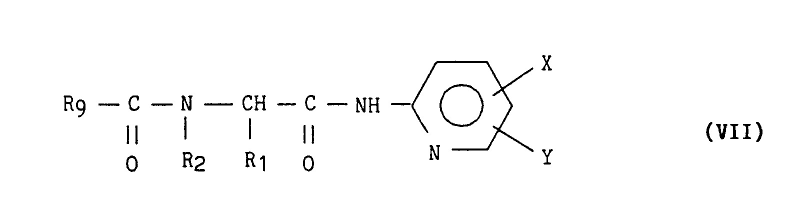
dans laquelle R, R₂, R₉, X et Y ont la même définition que précédemment cas particulier des dérivés de formule (I) pour lesquels A représente un un groupement 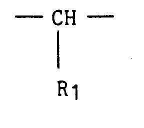 et R₃ un groupement -CO-R₉ avec R₁ et R₉ tels que définis précédemment,
qui, lorsque R₉ représente un groupement, 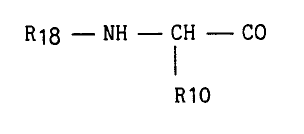 peut-être si on le désire, soumis à déprotection pour conduire à un dérivé de formule (XVII) 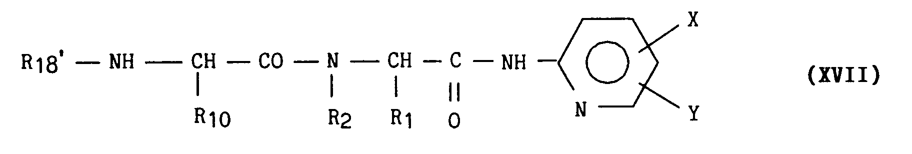
où R'18 représente un atome d'hydrogène ou un radical glycyle et R₁, R₂, R₁₀, X et Y ont la même définition que précédemment,
cas particulier des dérivés de formule (I) pour lesquels A représente un groupement 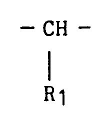 et R₃ un groupement 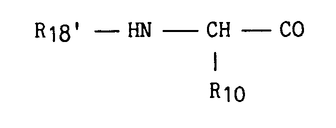
avec R₁ R_{18'} et R₁₀ ayant la même définition que précédemment
dérivés de formule (IV), (V), (VII) et (XVII) que l'on transforme en N oxyde par traitement par l'eau oxygénée et/ou dont on sépare éventuellement les isomères et que l'on salifie, si on le désire, par un acide ou une base pharmaceutiquement acceptable.

18. Procédé de synthèse des composés de formule (I) selon la revendication 1, pour lesquels A représente un groupement (CH₂)ₙCO avec n compris inclusivement entre 1 et 6, caractérisé en ce que l'on utilise comme matière première un dérivé de formule (II) : 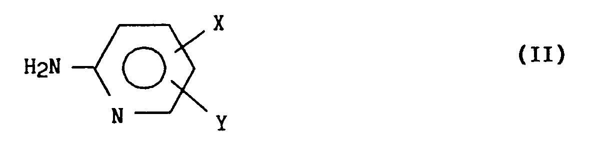
avec X et Y identiques ou différents représentant chacun un groupement alkyle,
que l'on condense
avec un dérivé de formule (VIII) : 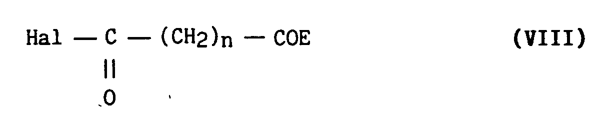
avec n ayant la même définition que précédemment Hal représentant un atome d'halogène et E représentant un groupement alkoxy, pour obtenir un dérivé de formule (IX) : 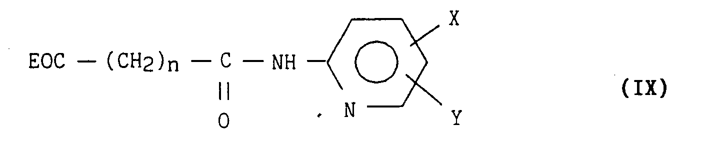
avec n, X, Y et E ayant la même définition que précédemment
qui, traité par un agent alcalin, donne un dérivé de formule (X) : 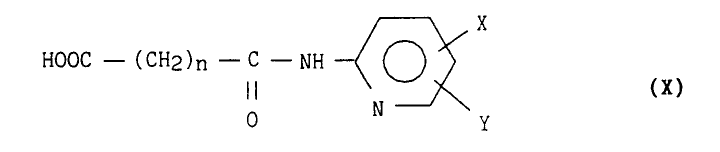
avec n, X et Y ayant la même définition que précédemment, que l'on traite :
- soit par un dérivé de formule (XI) : 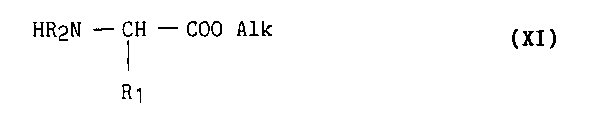
avec R₁ et R₂ ayant la même définition que précédemment et Alk représente un groupement alkyle,
pour conduire à un dérivé de formule (XII) : 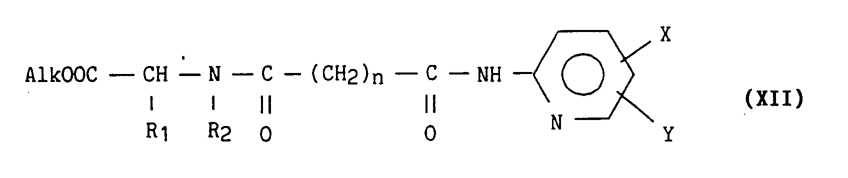 cas particulier de dérivé de formule (I) pour lequel A représente un un groupement (CH₂)ₙCO et R₃ un groupement 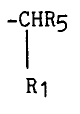 avec R₅ alkoxycarbonyle
et n, R₁ et R₂ ayant la même définition que précédemment,
dérivé de formule (XII) qui, par déprotection, conduit à un dérivé de formule (XIII) : 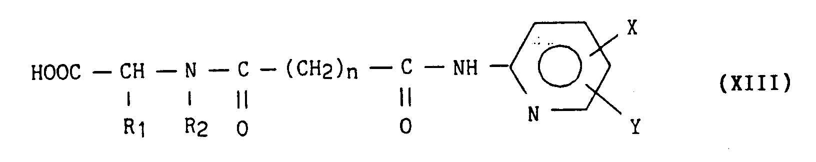 cas particulier des dérivés de formule (I) pour lesquels A représente un un groupement (CH₂)ₙCO et R₃ un groupement 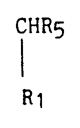 avec R₅ représentant ici un groupement carboxyle, n R₁ et R₂ ayant la même définition que précédémment,
dérivé de formule (XIII) que l'on peut traiter par un dérivé de formule (XIV) : 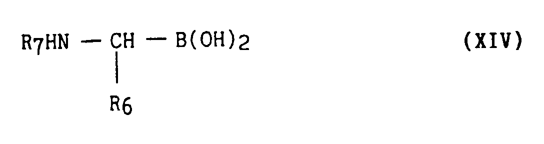 ou R₆ et R₇ ont la même définition que dans la formule (I),
éventuellement protégé pour conduire après éventuelle déprotection à un dérivé de formule (XV) : 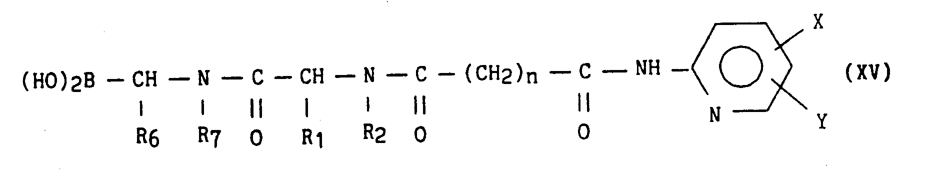 cas particulier des dérivés de formule (I) pour lesquels A représente un groupement -(CH₂)ₙCO- et R₃ un groupement 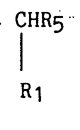 avec R₅ représentant un groupement 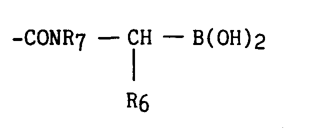
avec n, R₅, R₁, R₆, R₇ ayant la même définition que dans la formule (I),
- soit directement par un dérivé de formule (XIV) éventuellement protégé pour conduire après éventuelle déprotection à un dérivé de formule (XVI) : 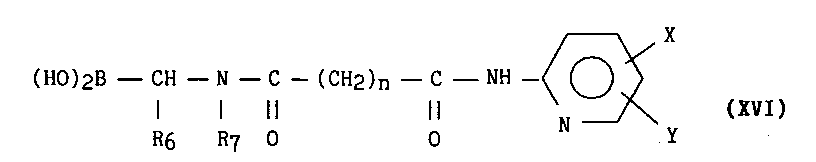
cas particulier de dérivé de formule (I) pour lequel A représente un
un groupement 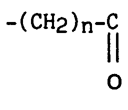 et R₃ un groupement 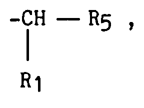 R₅ représentant ici un groupement B(OH)₂ et R₆ ayant la définition préalablement donnée pour R₁, R₇ ayant la définition préalablement donnée pour R₂,
dérivé de formule (XII), (XIII), (XV) et (XVI) que l'on transforme en N oxyde par traitement par l'eau oxygénée et/ou dont on sépare éventuellement les isomères et que l'on salifie si on le désire par un acide ou une base pharmaceutiquement acceptable.

19. Composition pharmaceutique contenant comme principe actif au moins un composé selon l'une des revendications 1 à 16 en combinaison avec un ou plusieurs excipients ou véhicules inertes, non toxiques, pharmaceutiquement acceptables.

20. Composition pharmaceutique selon la revendication 19 contenant au moins un principe actif selon l'une des revendications 1 à 16 utilisables dans le traitement des troubles inflammatoires, du psoriasis dans les hypercholestérolémies et hypertriglycéridémies, ainsi que dans le psoriasis.

## Claims

1. Compounds of the general formula (I): 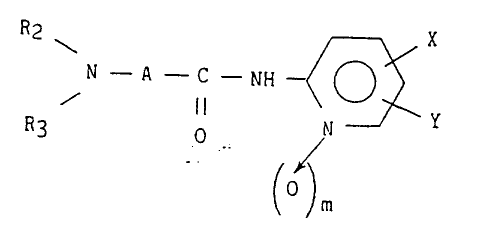 wherein :
m represents 0 or 1,
X and Y, which are identical or different, each represents an alkyl group,
A represents :
1) - a grouping 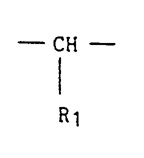
in which R₁ represents :
- a hydrogen atom,
- an alkyl group,
- an alkyl group substituted by an amino group,
- an alkylthioalkyl group,
- an alkyl group substituted by an amino group which is itself substituted on the amino group by a group selected from tert-butoxycarbonyl, benzyloxycarbonyl and fluorenylmethoxycarbonyl,
- a phenyl or phenylalkyl group,
- a phenyl or phenylalkyl group substituted on the aromatic nucleus by an alkyl, hydroxy, alkoxy or trifluoromethyl group or by a halogen atom,
and in that case (where A represents a -CH(R₁)- grouping) :
R₂ represents a hydrogen atom or R₂ forms together with R₁ and the CH-N- chain member carrying them a mono- or bi-cyclic carbon-containing system containing the -CH-N- chain member, each ring having 5 or 6 ring members and it being possible for each ring to be saturated or unsaturated,
and then
R₃ represents :
- a hydrogen atom,
- a tert-butoxycarbonyl, benzyloxycarbony or fluorenylmethoxycarbonyl group,
- a benzoyl group,
- a benzoyl group substituted by an alkyl, alkoxy, hydroxy or trifluoromethyl group or by a halogen atom,
- a grouping 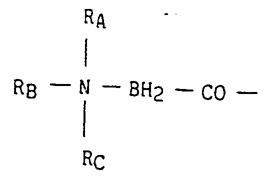 wherein R_{A}, R_{B} and R_{C},
which are identical are different, each represents a hydrogen atom or an alkyl group, or two of the groups R_{A}, R_{B} and R_{C} form together with the nitrogen atom carrying them a heterocyclic system selected from pyrrolidine, piperidine, azepine and morpholine, the third of the groups R_{A}, R_{B} and R_{C} then being either a hydrogen atom or an alkyl group,
- a grouping 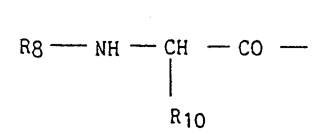
wherein R₈ represents hydrogen, tert-butoxycarbonyl, benzyloxycarbonyl, fluorenylmethoxycarbonyl, glycyl or glycyl substituted by a tert-butoxycarbonyl, benzyloxycarbonyl or fluorenylmethoxycarbonyl group and R₁₀ represents hydrogen; alkyl; alkyl substituted by an amino group; alkyl substituted by an amino group which is itself substituted by a tert-butoxycarbonyl, benzyloxycarbonyl or fluorenylmethoxycarbonyl group; a phenyl or phenylalkyl group, or a phenyl or phenylalkyl group substituted on the phenyl nucleus by an alkyl, hydroxy, alkoxy or trifluoromethyl group or by a halogen atom,
2) - a grouping 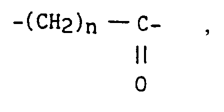 the 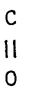 group of the radical A being bonded to the 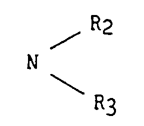 grouping of the compound of formula (I) and n being an integer of from 1 to 6 inclusive,
and in that case R₃ represents a grouping 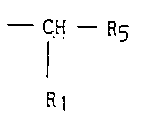
R₁ and R₂ being as defined above and R₅ representing a B(OH)₂, carboxy, alkoxycarbonyl or 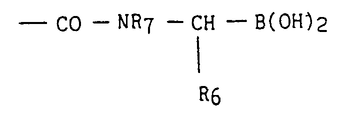 grouping in which
R₆ and R₁, which are identical or different, each has the definition given above for R₁, and R₇ has the definition given above for R₂ and is identical to or different from R₂,
their isomers, epimers, enantiomers and diastereoisomers, and also the addition salts thereof with a pharmaceutically acceptable acid or base,
there being understood by "alkyl" and "alkoxy" linear or branched groups having from 1 to 6 carbon atoms.

2. Compounds according to claim 1 of the general formula (I): 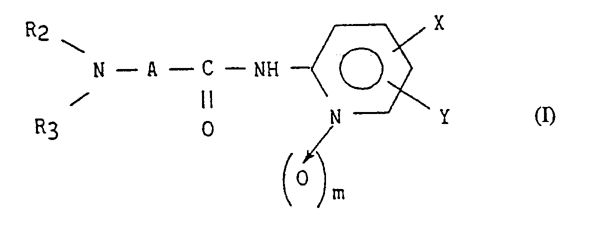 wherein :
m represents 0 or 1,
X and Y, which are identical or different, each represents an alkyl group, and
A represents a grouping 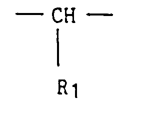
in which R₁ represents
- a hydrogen atom,
- an alkyl group,
- an alkyl group substituted by an amino group,
- an alkylthioalkyl group,
- an alkyl group substituted by an amino group which is itself substituted on the amino group by a group selected from tert-butoxycarbonyl, benzyloxycarbonyl and fluorenylmethoxycarbonyl,
- a phenyl or phenylalkyl group,
- a phenyl or phenylalkyl group substituted on the aromatic nucleus by an alkyl, hydroxy, alkoxy or trifluoromethyl group or by a halogen atom,
R₂ represents a hydrogen atom or R₂ forms together with R₁ and the CH-N- chain member carrying them a mono- or bi-cyclic carbon-containing system containing the -CH-N- chain member, each ring having 5 or 6 ring members and it being possible for each ring to be saturated or unsaturated,
and
R₃ represents :
- a hydrogen atom,
- a tert-butoxycarbonyl, benzyloxycarbonyl or fluorenylmethoxycarbonyl group,
- a benzoyl group,
- a benzoyl group substituted by an alkyl, alkoxy, hydroxy or trifluoromethyl group or by a halogen atom,
- a grouping 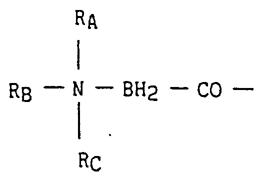 wherein R_{A}, R_{B} and R_{C},
which are identical are different, each represents a hydrogen atom or an alkyl group, or two of the groups R_{A}, R_{B} and R_{C} form together with the nitrogen atom carrying them a heterocyclic system selected from pyrrolidine, piperidine, azepine and morpholine, the third of the groups R_{A}, R_{B} and R_{C} then being either a hydrogen atom or an alkyl group,
their isomers, epimers, enantiomers and diastereoisomers, and also the addition salts thereof with a pharmaceutically acceptable acid or base,
there being understood by "alkyl" and "alkoxy" linear or branched groups having from 1 to 6 carbon atoms.

3. Compounds of the general formula (I) according to claim 1 : 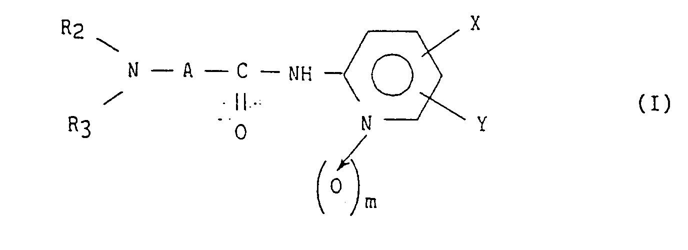 wherein :
m represents 0 or 1,
X and Y, which are identical or different, each represents an alkyl group,
A represents a grouping 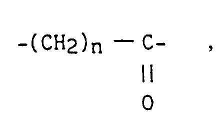 the 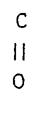 group of the radical A being bonded to the grouping 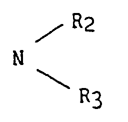 of the compound of formula (I) and n being an integer of from 1 to 6 inclusive,
R₃ represents a grouping 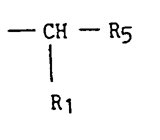
in which R₁ represents :
- a hydrogen atom,
- an alkyl group,
- an alkyl group substituted by an amino group,
- an alkylthioalkyl group,
- an alkyl group substituted by an amino group which is itself substituted on the amino group by a group selected from tert-butoxycarbonyl, benzyloxycarbonyl and fluorenylmethoxycarbonyl,
- a phenyl or phenylalkyl group,
- a phenyl or phenylalkyl group substituted on the aromatic nucleus by an alkyl, hydroxy, alkoxy or trifluoromethyl group or by a halogen atom,
and R₂ represents a hydrogen atom or R₂ forms together with R₁ and the CH-NR₃- chain member carrying them a mono- or bi-cyclic carbon-containing system containing the -CH-N(R₃)- chain member, each ring having 5 or 6 ring members and it being possible for each ring to be saturated or unsaturated,
R₅ representing a B(OH)₂, carboxy, alkoxycarbonyl 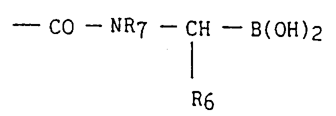 or grouping in which R₆ and R₁, which are identical or different, each has the definition given above for R₁, and R₇ has the definition given above for R₂ and is identical to or different from R₂,
their isomers, epimers, enantiomers and diastereoisomers, and the addition salts thereof with a pharmaceutically acceptable acid or base.

4. Compounds of the general formula (I/A) according to claim 1 : 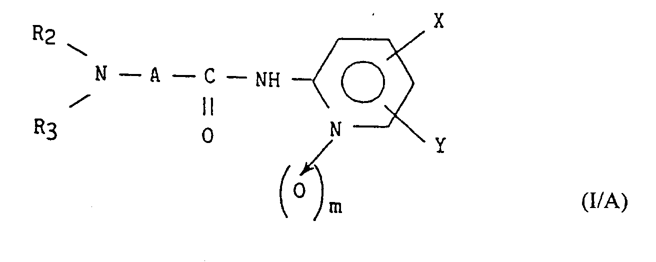
wherein m is 0 or 1, X and Y, which are identical or different, each represents an alkyl group
and A represents a grouping 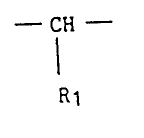
in which R₁ represents :
- a hydrogen atom,
- an alkyl group substituted by an amino group,
- an alkylthioalkyl group,
- an alkyl group substituted by an amino group which is itself substituted on the amino group by a group selected from tert-butoxycarbonyl, benzyloxycarbonyl and fluorenylmethoxycarbonyl,
- a phenyl or phenylalkyl group,
- a phenyl or phenylalkyl group substituted on the aromatic nucleus by an alkyl, hydroxy, alkoxy or trifluoromethyl group or by a halogen atom,
and R₃ represents :
- a grouping 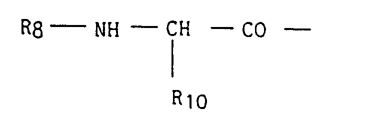
in which R₈ represents hydrogen, tert-butoxycarbonyl, benzyloxycarbonyl or fluorenylmethoxycarbonyl and R₁₀ represents hydrogen; alkyl; alkyl substituted by an amino group; alkyl substituted by an amino group which is itself substituted by a tert-butoxycarbonyl, benzyloxycarbonyl or fluorenylmethoxycarbonyl group; a phenyl or phenylalkyl group, or a phenyl or phenylalkyl group substituted on the phenyl nucleus by an alkyl, hydroxy, alkoxy or trifluoromethyl group or by a halogen atom,
their isomers, epimers, enantiomers and diastereoisomers, and the addition salts thereof with a pharmaceutically acceptable acid or base.

5. Compounds of formula (I/A) according to claim 1 : 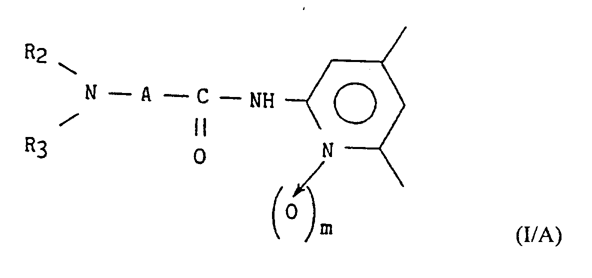
wherein m represents 0 or 1, A represents a grouping 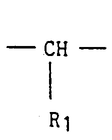 in which R₁ is selected from hydrogen, alkyl and benzyl, R₂ represents hydrogen, benzyloxycarbonyl or tert-butoxycarbonyl, and R₃ represents : 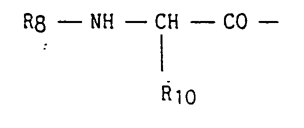
in which R₈ represents hydrogen, tert-butoxycarbonyl, benzyloxycarbonyl, fluorenylmethoxycarbonyl, glycyl or glycyl substituted by a tert-butoxycarbonyl, benzyloxycarbonyl or fluorenylmethoxycarbonyl group and R₁₀ represents hydrogen; alkyl; alkyl substituted by an amino group; alkyl substituted by an amino group which is itself substituted by a tert-butoxycarbonyl, benzyloxycarbonyl or fluorenylmethoxycarbonyl group; a phenyl or phenylalkyl group, or a phenyl or phenylalkyl group substituted on the phenyl nucleus by an alkyl, hydroxy, alkoxy or trifluoromethyl group or by a halogen atom,
their isomers, epimers, enantiomers and diastereoisomers, and the addition salts thereof with a pharmaceutically acceptable acid or base.

6. Compounds of formula (I/A) according to claim 1 : 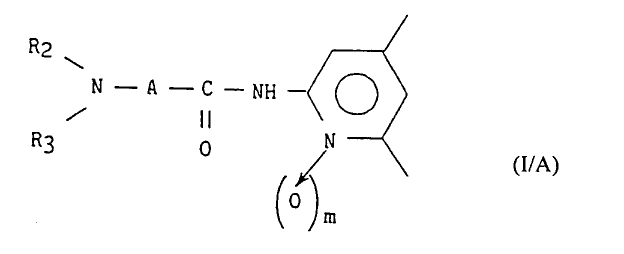
wherein m represents 0 or 1, A represents a grouping 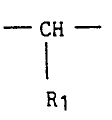 in which R₁ is selected from hydrogen, alkyl and benzyl, R₂ represents hydrogen and R₃ represents a grouping 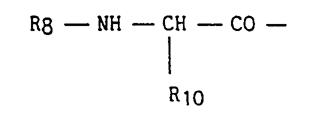
in which R₈ represents hydrogen, tert-butoxycarbonyl, benzyloxycarbonyl, fluorenylmethoxycarbonyl, glycyl or glycyl substituted by a tert-butoxycarbonyl, benzyloxycarbonyl or fluorenylmethoxycarbonyl group and R₁₀ represents hydrogen; alkyl; alkyl substituted by an amino group; alkyl substituted by an amino group which is itself substituted by a tert-butoxycarbonyl, benzyloxycarbonyl or fluorenylmethoxycarbonyl group; a phenyl or phenylalkyl group, or a phenyl or phenylalkyl group substituted on the phenyl nucleus by an alkyl, hydroxy, alkoxy or trifluoromethyl group or by a halogen atom,
their isomers, epimers, enantiomers and diastereoisomers, and the addition salts thereof with a pharmaceutically acceptable acid or base.

7. Compound according to claim 1, which is N-2-benzyloxycarbonyl-N-(4,6-dimethylpyridin-2-yl)prolinamide, its N-oxide, the isomers thereof, and also the addition salts thereof with a pharmaceutically acceptable acid or base.

8. Compound according to claim 1, which is N-2-benzyloxycarbonyl-N-(4,6-dimethylpyridin-2-yl)glycinamide, its N-oxide, and also the addition salts thereof with a pharmaceutically acceptable acid or base.

9. Compound according to claim 1, which is N-2-(3-fluorobenzoyl)-N-(4,6-dimethylpyridin-2-yl)glycinamide, its N-oxide, and also the addition salts thereof with a pharmaceutically acceptable acid or base.

10. Compounds of formula I according to claim 1 wherein A represents a grouping : 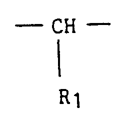 and R₃ represents a grouping 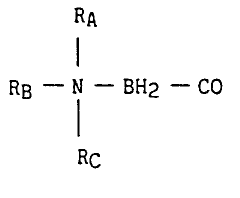 wherein R_{A}, R_{B} and R_{C}, which are identical are different, each represents a hydrogen atom or an alkyl group, or two of the groups R_{A}, R_{B} and R_{C} form together with the nitrogen atom carrying them a heterocyclic system selected from pyrrolidine, piperidine, azepine and morpholine, the third of the groups R_{A}, R_{B} and R_{C} then being either a hydrogen atom or an alkyl group and being identical or different, their isomers and also their N-oxides, and the addition salts thereof with a pharmaceutically acceptable acid or base.

11. Compound according to claim 1, which is ethyl N-{[1,4-dioxo-4-(4,6-dimethylpyridin-2-ylamino)]butyl}glycinate, and also its N-oxide, and the addition salts thereof with a pharmaceutically acceptable acid or base.

12. Compound according to claim 1, which is {[N-(4,6-dimethylpyridin-2-yl)-2-leucinamido]carbonyl}trimethylaminoboron dihydride, and also its N-oxide, and the addition salts thereof with a pharmaceutically acceptable acid or base.

13. Compound according to claim 1, which is {[N-(4,6-dimethylpyridin-2-yl)-2-methioninamido]carbonyl}trimethylaminoboron dihydride, its N-oxide, its isomers, and also the addition salts thereof with a pharmaceutically acceptable acid or base.

14. Compound according to claim 1, which is N-(4,6-dimethylpyridin-2-yl)glycinamide, and also its N-oxide, and the addition salts thereof with a pharmaceutically acceptable acid or base.

15. Compound according to claim 1, which is N-(4,6-dimethylpyridin-2-yl)glycyl glycinamide, and also its N-oxide, and the addition salts thereof with a pharmaceutically acceptable acid or base.

16. Compound according to claim 1, which is N-(4,6-dimethylpyridin-2-yl)glycylgylcyl glycinamide, and also its N-oxide, and the addition salts thereof with a pharmaceutically acceptable acid or base.

17. Process for the synthesis of compounds of formula (I) according to claim 1, wherein :
A represents a grouping 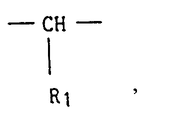 characterised in that the starting material used is a compound of formula (II) : 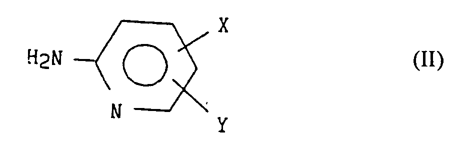
wherein X and Y, which are identical or different, each represents an alkyl group, which compound is condensed with a compound of formula (III) : 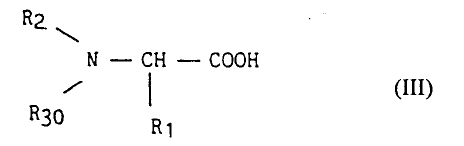
wherein R₁ and R₂ are as defined for formula (I),
and R₃₀ represents a tert-butoxycarbonyl, benzyloxycarbonyl or fluorenylmethoxycarbonyl group or a grouping 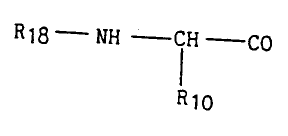
in which R₁₀ is as defined above and R₁₈ represents a tert-butoxycarbonyl, benzyloxycarbonyl or fluorenylmethoxycarbonyl group,
to yield a compound of formula (IV) : 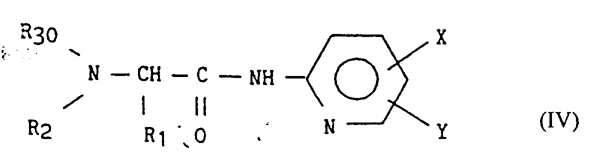 a particular case of compounds of formula (I) in which A represents a grouping 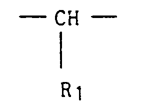 and R₃ represents a grouping R₃₀ as defined above,
which compound of formula (IV) may then be deprotected to yield a compound of formula (V) : 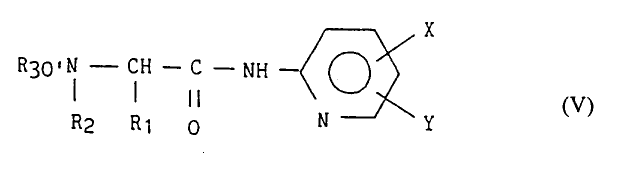
wherein R₁, R₂, X and Y are as defined above and R_{30'} represents either a hydrogen atom or a grouping 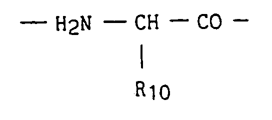
in which R₁₀ is as defined above,
a particular case of compounds of formula (I) wherein R₃ represents a group R_{30'} and A represents a grouping 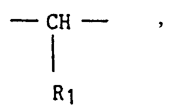 R₁ and R_{30'} being as defined above,
which compound of formula (V), when R_{30'} represents a hydrogen atom, may be condensed with a compound of formula (VI)
R₉COOH (VI)
in which R₉ represents :
- a phenyl group,
- a phenyl group substituted by an alkyl, alkoxy, hydroxy or trifluoromethyl group or by a halogen atom,
- a grouping 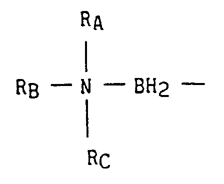 in which R_{A}, R_{B} and R_{C} are as defined above,
- a grouping 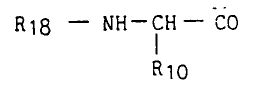
in which R₁₈ represents tert-butoxycarbonyl, benzyloxycarbonyl, fluorenylmethoxycarbonyl, glycyl or glycyl substituted by a tert-butoxycarbonyl, benzyloxycarbonyl or fluorenylmethoxycarbonyl group and R₁₀ is as defined above, to yield a compound of formula (VII) : 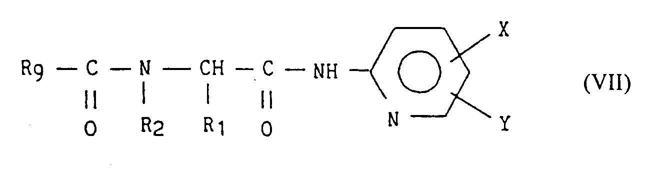
wherein R, R₂, R₉. X and Y are as defined above, a particular case of compounds of formula (I) wherein A represents a grouping 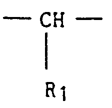 and R₃ represents a grouping -CO-R₉ in which R₁ and R₉ are as defined above,
which compound, when R₉ represents a grouping 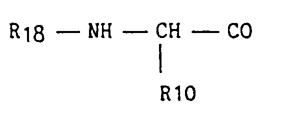 may, if desired, be subjected to deprotection to yield a compound of formula (XVII) 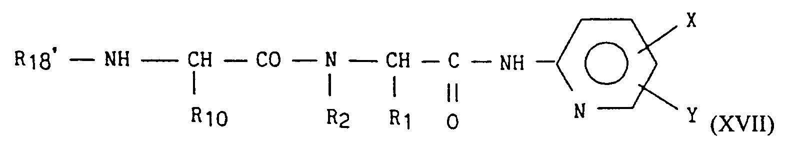
wherein R_{18'} represents a hydrogen atom or a glycyl radical and R₁, R₂, R₁₀, X and Y are as defined above,
a particular case of compounds of formula (I) wherein A represents a grouping 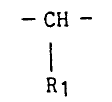 and R₃ represents a grouping 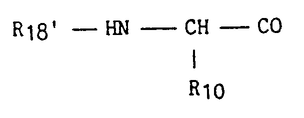 in which R₁, R_{18'} and R₁₀ are as defined above,
which compounds of formula (IV), (V), (VII) and (XVII) are converted into N-oxides by treatment with hydrogen peroxide and/or, where appropriate, are separated into their isomers and, if desired, are converted into salts with a pharmaceutically acceptable acid or base.

18. Process for the synthesis of compounds of formula (I) according to claim 1, wherein A represents a grouping (CH₂)ₙCO in which n is from 1 to 6 inclusive, characterised in that the starting material used is a compound of formula (II) : 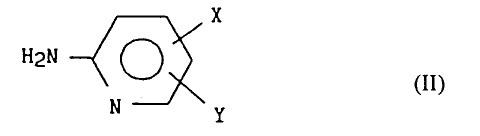
wherein X and Y, which are identical or different, each represents an alkyl group, which compound is condensed
with a compound of formula (VIII) : 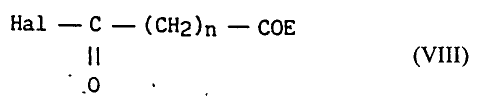
wherein n is as defined above, Hal represents a halogen atom and E represents an alkoxy group, to obtain a compound of formula (IX) : 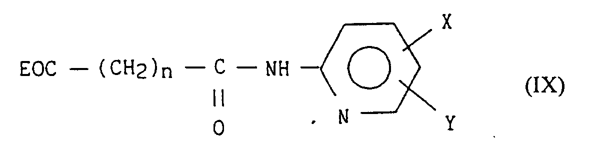
wherein n, X, Y and E are as defined above,
which, when treated with an alkaline agent, yields a compound of formula (X) : 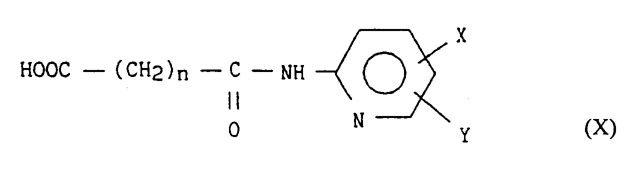
wherein n, X and Y are as defined above,
which is treated :
- either with a compound of formula (XI) : 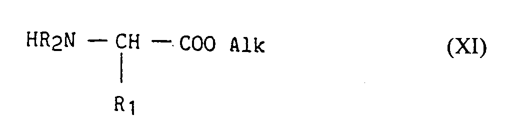
wherein R₁ and R₂ are as defined above and Alk represents an alkyl group,
to yield a compound of formula (XII) : 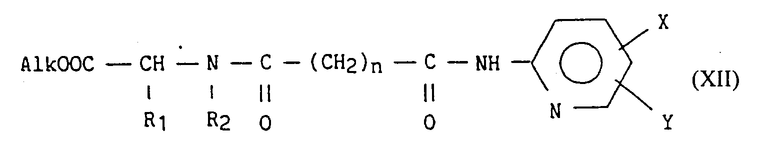 a particular case of a compound of formula (I) wherein A represents a grouping (CH₂)ₙCO and R₃ represents a grouping 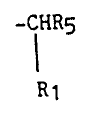 in which R₅ represents alkoxycarbonyl, and n, R₁ and R₂ are as defined above,
which compound of formula (XII), on deprotection, yields a compound of formula (XIII) : 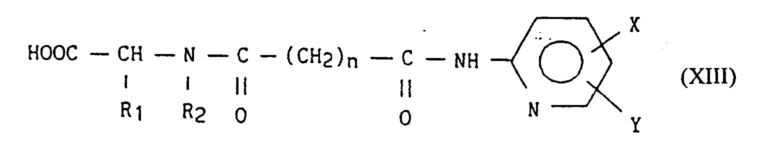 a particular case of compounds of formula (I) wherein A represents a grouping (CH₂)ₙCO and R₃ represents a grouping 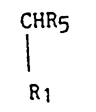 in which R₅ in this case represents a carboxyl group, and n, R₁ and R₂ are as defined above,
which compound of formula (XIII) may be treated with a compound of formula (XIV) : 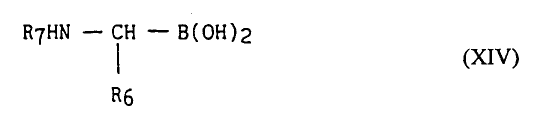 wherein R₆ and R₇ are as defined for formula (I),
which has been protected where necessary, to yield, after deprotection where required, a compound of formula (XV) : 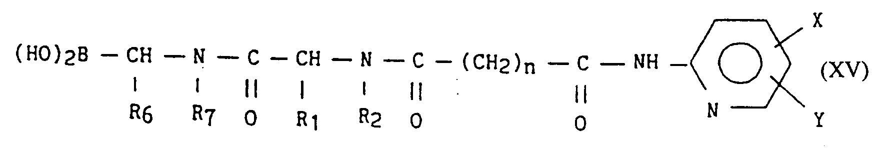 a particular case of compounds of formula (I) wherein A represents a grouping -(CH₂)ₙCO- and R₃ represents a grouping 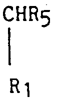 in which R₅ represents a grouping 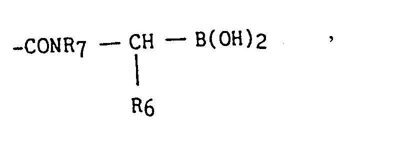
n, R₅, R₁, R₆ and R₇ being as defined for formula (I),
- or directly with a compound of formula (XIV), which has been protected where necessary, to yield, after deprotection where required, a compound of formula (XVI) : 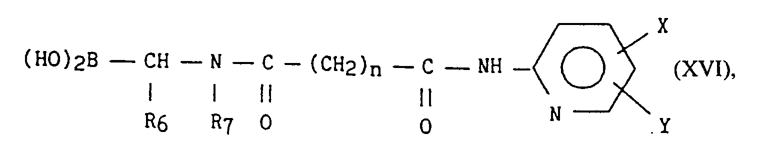
a particular case of a compound of formula (I) wherein A represents a grouping 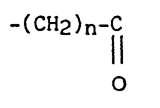 and R₃ represents a grouping 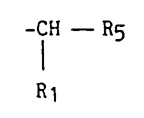 in which R₅ in this case represents a B(OH)₂ group, and wherein R₆ has the definition given above for R₁, and R₇ has the definition given above for R₂,
which compounds of formula (XII), (XIII), (XV) and (XVI) are converted into the N-oxides by treatment with hydrogen peroxide and/or, where applicable, separated into their isomers and, if desired, converted into salts with a pharmaceutically acceptable acid or base.

19. Pharmaceutical composition comprising as active ingredient at least one compound according to any one of claims 1 to 16 in combination with one or more pharmaceutically acceptable, inert, non-toxic excipients or carriers.

20. Pharmaceutical composition according to claim 19, comprising at least one active ingredient according to any one of claims 1 to 16 utilisable in the treatment of inflammatory disorders, of psoriasis in hypercholesterolaemia and hypertriglyceridaemia, and also in psoriasis.

## Patentansprüche

1. Verbindungen der allgemeinen Formel (I): 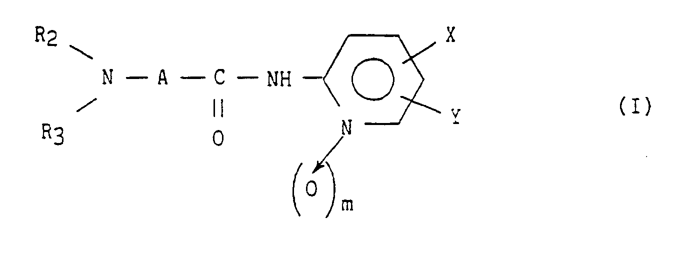 in der:
m 0 oder 1,
X und Y, die gleichartig oder verschieden sein können, jeweils eine Alkylgruppe,
A:
1) - eine Gruppe 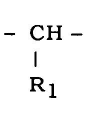
in der R₁:
- ein Wasserstoffatom,
- eine Alkylgruppe,
- eine Alkylgruppe, die durch eine Amingruppe substituiert ist,
- eine Alkylthioalkylgruppe,
- eine Alkylgruppe, die durch eine Amingruppe substituiert ist, die ihrerseits an der Aminogruppe durch eine tert.-Butoxycarbonylgruppe, Benzyloxycarbonylgruppe oder Fluorenylmethyloxycarbonylgruppe substituiert ist,
- eine Phenylgruppe oder Phenylalkylgruppe,
- eine Phenylgruppe oder Phenylalkylgruppe, die am aromatischen Kern durch eine Alkylgruppe, Hydroxylgruppe, Alkoxygruppe, Trifluormethylgruppe oder ein Halogenatom substituiert ist,
und in diesem Fall, wenn A eine Gruppe -CH(R₁)- bedeutet:
R₂ ein Wasserstoffatom darstellt oder R₂ zusammen mit R₁ und der sie tragenden CH-N-Kette ein mono- oder bicyclisches Kohlenstoffsystem bildet, welches die Kette -CH-N- aufweist, wobei jeder Ring 5 oder 6 Ringglieder aufweist und gesättigt oder ungesättigt sein kann,
und in diesem Fall
R₃:
- ein Wasserstoffatom,
- eine tert.-Butoxycarbonylgruppe, Benzyloxycarbonylgruppe oder Fluorenylmethyloxycarbonylgruppe,
- eine Benzoylgruppe,
- eine Benzoylgruppe, die durch eine Alkylgruppe, Alkoxygruppe, Hydroxylgruppe, Trifluormethylgruppe oder ein Halogenatom substituiert ist, - eine Gruppe 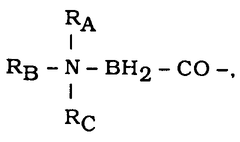 worin R_{A}, R_{B} und R_{C}, die gleichartig oder verschieden sein können, ein Wasserstoffatom oder eine Alkylgruppe darstellen oder zwei der Gruppen R_{A}, R_{B}, R_{C} mit dem sie tragenden Stickstoffatom ein heterocyclisches System bilden ausgewählt aus Pyrrolidin, Piperidin, Azepin oder Morpholin, wobei die dritte Gruppe der Reste R_{A}, R_{B} und R_{C} dann ein Wasserstoffatom oder eine Alkylgruppe darstellt,
- eine Gruppe 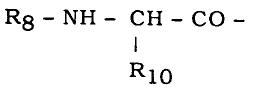
in der R₈ ein Wasserstoffatom, eine tert.-Butoxycarbonylgruppe, eine Benzyloxycarbonylgruppe, eine Fluorenylmethyloxycarbonylgruppe, eine Glycylgruppe, eine Glycylgruppe, die durch eine tert.-Butoxycarbonylgruppe, Benzyloxycarbonylgruppe oder Fluorenylmethyloxycarbonylgruppe substituiert ist, und R₁₀ ein Wasserstoffatom, eine Alkylgruppe, eine Alkylgruppe, die durch eine Aminogruppe substituiert ist, eine Alkylgruppe, die durch eine Aminogruppe substituiert ist, die ihrerseits durch eine tert.-Butoxycarbonylgruppe, Benzyloxycarbonylgruppe oder Fluorenylmethyloxycarbonylgruppe substituiert ist, eine Phenylgruppe oder eine Phenylalkylgruppe, eine Phenylgruppe oder eine Phenylalkylgruppe, die am Phenylkern durch eine Alkylgruppe, Hydroxylgruppe, Alkoxygruppe, Trifluormethylgruppe oder ein Halogenatom substituiert ist, darstellen,
2) - eine Gruppe 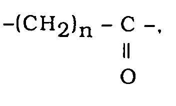 wobei die Gruppe 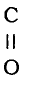 des Rests A an die Gruppe 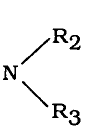 des Derivats der Formel (I) gebunden ist, wobei n eine ganze Zahl von 1 bis 6 einschließlich darstellt,
und in diesem Fall R₃ eine Gruppe 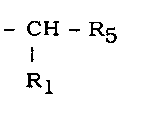
darstellt, in der R₁ und R₂ die oben angegebenen Bedeutungen besitzen und R₅ eine Gruppe B(OH)₂, eine Carboxylgruppe, eine Alkoxycarbonylgruppe oder 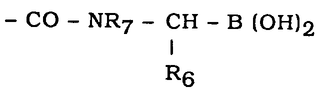 darstellt,
worin R₆ und R₁, die gleichartig oder verschieden sein können, jeweils die oben angegebenen Bedeutungen von R₁ besitzen, R₇ die oben angegebenen Bedeutungen von R₂ aufweist und mit R₂ identisch oder davon verschieden ist, bedeuten, deren Isomere, Epimere, Enantiomere, Diastereoisomere sowie deren Additionssalze mit einer pharmazeutisch annehmbaren Säure oder Base,
wobei es sich versteht, daß es sich bei den Alkyl- oder Alkoxgruppen um geradkettige oder verzweigte Gruppen mit 1 bis 6 Kohlenstoffatomen handelt.

2. Verbindungen nach Anspruch 1 der allgemeinen Formel (I): 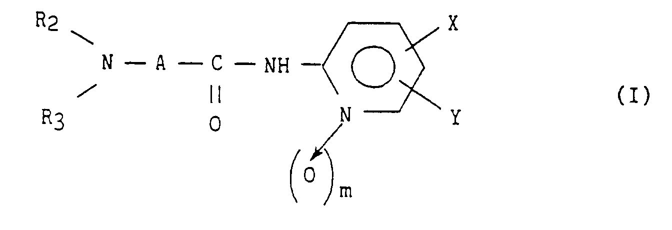 in der:
m 0 oder 1,
X und Y, die gleichartig oder verschieden sein können, jeweils eine Alkylgruppe darstellen und
A - eine Gruppe 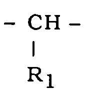 bedeutet,
in der R₁:
- ein Wasserstoffatom,
- eine Alkylgruppe,
- eine Alkylgruppe, die durch eine Amingruppe substituiert ist,
- eine Alkylthioalkylgruppe,
- eine Alkylgruppe, die durch eine Amingruppe substituiert ist, die ihrerseits an der Aminogruppe durch eine tert.-Butoxycarbonylgruppe, Benzyloxycarbonylgruppe oder Fluorenylmethyloxycarbonylgruppe substituiert ist,
- eine Phenyl- oder Phenylalkylgruppe,
- eine Phenyl- oder Phenylalkylgruppe, die am aromatischen Kern durch eine Alkylgruppe, Hydroxylgruppe, Alkoxygruppe, Trifluormethylgruppe oder ein Halogenatom substituiert ist,
R₂ ein Wasserstoffatom oder R₂ zusammen mit R₁ und der sie tragenden Kette CH-N- ein mono- oder bicyclisches Kohlenstoffsystem bildet, welches die Kette -CH-N- aufweist, wobei jeder Ring 5 oder 6 Ringglieder aufweist und gesättigt oder ungesättigt sein kann, und
R₃:
- ein Wasserstoffatom,
- eine tert.-Butoxycarbonylgruppe, Benzyloxycarbonylgruppe oder Fluorenylmethyloxycarbonylgruppe,
- eine Benzoylgruppe,
- eine Benzoylgruppe, die durch eine Alkylgruppe, Alkoxygruppe, Hydroxylgruppe, Trifluormethylgruppe oder ein Halogenatom substituiert ist,
- eine Gruppe 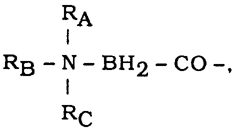 worin R_{A}, R_{B} und R_{C}, die gleichartig oder verschieden sein können, ein Wasserstoffatom oder eine Alkylgruppe darstellen oder zwei der Gruppen R_{A}, R_{B}, R_{C} mit dem sie tragenden Stickstoffatom ein heterocyclisches System ausgewählt aus Pyrrolidin, Piperidin, Azepin oder Morpholin bilden, wobei die dritte Gruppe der Reste R_{A}, R_{B} und R_{C} dann ein Wasserstoffatom oder eine Alkylgruppe darstellt, bedeuten,
deren Isomere, Epimere, Enantiomere, Diastereoisomere sowie deren Additionssalze mit einer pharmazeutisch annehmbaren Säure oder Base,
wobei unter Alkyl- oder Alkoxygruppen geradkettige oder verzweigte Gruppen mit 1 bis 6 Kohlenstoffatomen zu verstehen sind.

3. Verbindungen der allgemeinen Formel (I) nach Anspruch 1: 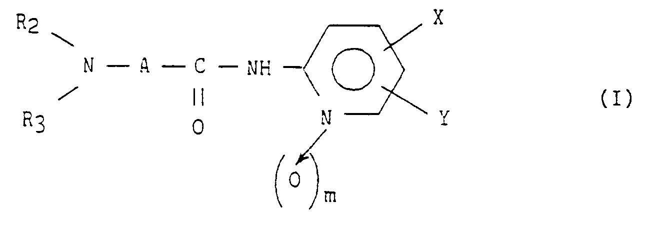 in der:
m 0 oder 1,
X und Y, die gleichartig oder verschieden sein können, jeweils eine Alkylgruppe und A eine Gruppe 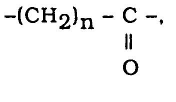 wobei die Gruppe 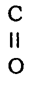 des Rests A an die Gruppe 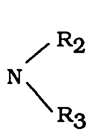 des Derivats der Formel (I) gebunden ist, wobei n eine ganze Zahl von 1 bis 6 einschließlich darstellt,
R₃ eine Gruppe 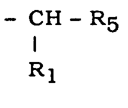 darstellt,
R₁:
- ein Wasserstoffatom,
- eine Alkylgruppe,
- eine Alkylgruppe, die durch eine Amingruppe substituiert ist,
- eine Alkylthioalkylgruppe,
- eine Alkylgruppe, die durch eine Amingruppe substituiert ist, die ihrerseits an der Aminogruppe durch eine tert.-Butoxycarbonylgruppe, Benzyloxycarbonylgruppe oder Fluorenylmethyloxycarbonylgruppe substituiert ist,
- eine Phenyl- oder Phenylalkylgruppe,
- eine Phenyl- oder Phenylalkylgruppe, die am aromatischen Kern durch eine Alkylgruppe, Hydroxylgruppe, Alkoxygruppe, Trifluormethylgruppe oder durch ein Halogenatom substituiert ist,
R₂ ein Wasserstoffatom oder R₂ zusammen mit R₁ und der sie tragenden Kette CH-NR₃ ein mono- oder bicyclisches Kohlenstoffsystem bildet, welches die Kette -CH-N(R₃) einschließt, wobei jeder Zyklus 5 oder 6 Ringglieder aufweist und gesättigt oder ungesättigt sein kann,
und R₅ eine Gruppe B(OH)₂, eine Carboxylgruppe, eine Alkoxycarbonylgruppe oder 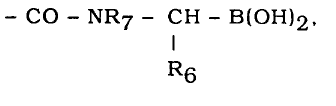 worin R₆ und R₁, die gleichartig oder verschieden sein können, jeweils die oben angegebenen Bedeutungen von R₁ besitzen und R₇ die oben angegebenen Bedeutungen von R₂ aufweist und mit R₂ identisch oder davon verschieden sein kann, bedeuten,
deren Isomere, Epimere, Enantiomere, Diastereoisomere sowie deren Additionssalze mit einer pharmazeutisch annehmbaren Säure oder Base.

4. Verbindungen der allgemeinen Formel (I/A) nach Anspruch 1: 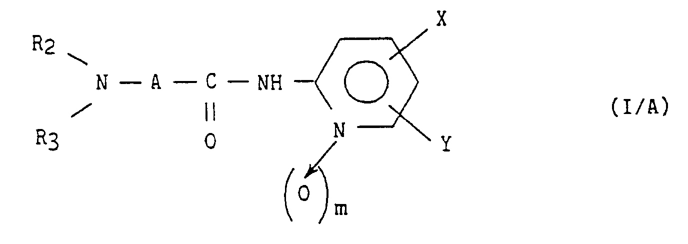
in der m 0 oder 1, X und Y, die gleichartig oder verschieden sein können, jeweils eine Alkylgruppe und A eine Gruppe 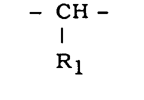
R₁:
- ein Wasserstoffatom
- eine Alkylgruppe, die durch eine Amingruppe substituiert ist,
- eine Alkylthioalkylgruppe,
- eine Alkylgruppe, die durch eine Amingruppe substituiert ist, die ihrerseits an der Aminogruppe durch eine tert.-Butoxycarbonylgruppe, Benzyloxycarbonylgruppe oder Fluorenylmethyloxycarbonylgruppe substituiert ist,
- eine Phenyl- oder Phenylalkylgruppe,
- eine Phenyl- oder Phenylalkylgruppe, die am aromatischen Kern durch eine Alkylgruppe, Hydroxylgruppe, Alkoxygruppe, Trifluormethylgruppe oder ein Halogenatom substituiert ist,
und R₃:
- eine Gruppe 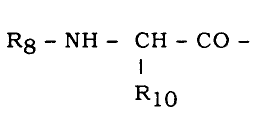
in der R₈ ein Wasserstoffatom, eine tert.-Butoxycarbonylgruppe, eine Benzyloxycarbonylgruppe oder eine Fluorenylmethyloxycarbonylgruppe und R₁₀ ein Wasserstoffatom, eine Alkylgruppe, eine Alkylgruppe, die durch eine Aminogruppe substituiert ist, eine Alkylgruppe, die durch eine Aminogruppe substituiert ist, die ihrerseits durch eine tert.-Butoxycarbonylgruppe, Benzyloxycarbonylgruppe oder Fluorenylmethyloxycarbonylgruppe substituiert ist, eine Phenyl- oder Phenylalkylgruppe, eine Phenyl- oder Phenylalkylgruppe, die am Phenylkern durch eine Alkylgruppe, Hydroxylgruppe, Alkoxygruppe, Trifluormethylgruppe oder ein Halogenatom substituiert ist, darstellen, bedeuten,
deren Isomere, Epimere, Enantiomere, Diastereoisomere sowie deren Additionssalze mit einer pharmazeutisch annehmbaren Säure oder Base.

5. Verbindungen der Formel (I/A) nach Anspruch 1: 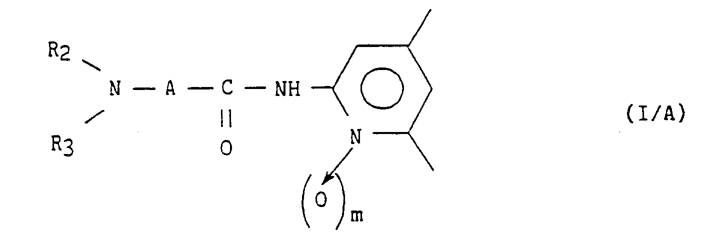
in der m 0 oder 1, A eine Gruppe 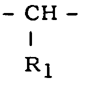 und R₁ aus Wasserstoff, Alkyl und Benzyl ausgewählt ist, R₂ ein Wasserstoffatom, eine Benzyloxycarbonylgruppe oder eine tert.-Butoxycarbonylgruppe und R₃: 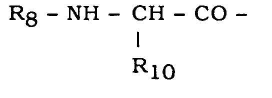
in der R₈ ein Wasserstoffatom, eine tert.-Butoxycarbonylgruppe, eine Benzyloxycarbonylgruppe, eine Fluorenylmethyloxycarbonylgruppe, eine Glycylgruppe, eine Glycylgruppe, die durch eine tert.-Butoxycarbonylgruppe, Benzyloxycarbonylgruppe oder Fluorenylmethyloxycarbonylgruppe substituiert ist, und R₁₀ ein Wasserstoffatom, eine Alkylgruppe, eine Alkylgruppe, die durch eine Aminogruppe substituiert ist, eine Alkylgruppe, die durch eine Aminogruppe substituiert ist, die ihrerseits durch eine tert.-Butoxycarbonylgruppe, Benzyloxycarbonylgruppe oder Fluorenylmethyloxycarbonylgruppe substituiert ist, eine Phenyl- oder Phenylalkylgruppe, eine Phenyl- oder Phenylalkylgruppe, die am Phenylkern durch eine Alkylgruppe, Hydroxylgruppe, Alkoxygruppe, Trifluormethylgruppe oder ein Halogenatom substituiert ist, darstellen, bedeuten,
deren Isomere, Epimere, Enantiomere, Diastereoisomere sowie deren Additionssalze mit einer pharmazeutisch annehmbaren Säure oder Base.

6. Verbindungen der Formel (I/A) nach Anspruch 1: 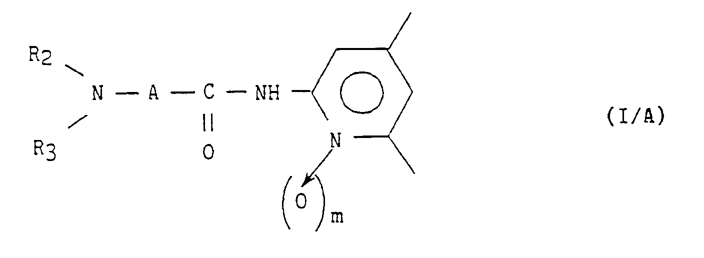
in der m 0 oder 1, A eine Gruppe 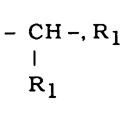 R₁ ausgewählt aus Wasserstoff, Alkyl oder Benzyl, R₂ Wasserstoff und R₃ eine Gruppe 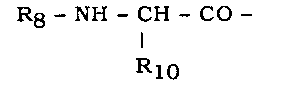
in der R₈ Wasserstoff, tert.-Butoxycarbonyl, Benzyloxycarbonyl, Fluorenylmethyloxycarbonyl, Glycyl, Glycyl substituiert durch eine tert.-Butoxycarbonylgruppe, Benzyloxycarbonylgruppe oder Fluorenylmethyloxycarbonylgruppe und R₁₀ Wasserstoff, Alkyl, Alkyl substituiert durch eine Aminogruppe, Alkyl substituiert durch eine Aminogruppe, die ihrerseits durch eine tert.-Butoxycarbonylgruppe, Benzyloxycarbonylgruppe oder Fluorenylmethyloxycarbonylgruppe substituiert ist, eine Phenyl- oder Phenylalkylgruppe, eine Phenyl- oder Phenylalkylgruppe, die am Phenylkern durch eine Alkylgruppe, Hydroxylgruppe, Alkoxygruppe, Trifluormethylgruppe oder ein Halogenatom substituiert ist, darstellen, bedeuten,
deren Isomere, Epimere, Enantiomere, Diastereoisomere sowie deren Additionssalze mit einer pharmazeutisch annehmbaren Säure oder Base.

7. Verbindung nach Anspruch 1, nämlich N-2-Benzyloxycarbonyl-N-(4,6-dimethylpyridin-2-yl)-prolinamid, dessen N-Oxid, dessen Isomere sowie dessen Additionssalze mit einer pharmazeutisch annehmbaren Säure oder Base.

8. Verbindung nach Anspruch 1, nämlich N-2-Benzyloxycarbonyl-N-(4,6-dimethylpyridin-2-yl)-glycinamid, dessen N-Oxid sowie dessen Additionssalze mit einer pharmazeutisch annehmbaren Säure oder Base.

9. Verbindung nach Anspruch 1, nämlich N-2-(3-Fluor)-benzoyl-N-(4,6-dimethylpyridin-2-yl)-glycinamid, dessen N-Oxid sowie dessen Additionssalze mit einer pharmazeutisch annehmbaren Säure oder Base.

10. Verbindung der Formel I nach Anspruch, worin A eine Gruppe: 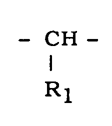 und R₃ eine Gruppe 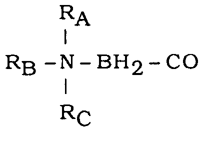 darstellen, worin R_{A}, R_{B} und R_{C}, die gleichartig oder verschieden sein können, ein Wasserstoffatom oder eine Alkylgruppe darstellen oder zwei der Gruppen R_{A}, R_{B} und R_{C} zusammen mit dem sie tragenden Stickstoffatom ein heterocyclisches System bilden ausgewählt aus Pyrrolidin, Piperidin, Azepin oder Morpholin, wobei der dritte Rest R_{A}, R_{B} oder R_{C} dann ein Wasserstoffatom oder eine Alkylgruppe bedeutet, deren Isomere sowie deren N-Oxide und deren Additionssalze mit einer pharmazeutisch annehmbaren Säure oder Base.

11. Verbindung nach Anspruch 1, nämlich N-{[1,4-Dioxo-4-(4,6-dimethylpyridin-2-yl)-amino]-butyl}-glycinsäureethylester, sowie dessen N-Oxid und dessen Additionssalze mit einer pharmazeutisch annehmbaren Säure oder Base.

12. Verbindung nach Anspruch 1, nämlich {[N-(4,6-Dimethylpyridin-2-yl)-2-leucinamido]-carbonyl}-trimethylamino-bordihydrid, sowie dessen N-Oxid und dessen Additionssalze mit einer pharmazeutisch annehmbaren Säure oder Base.

13. Verbindung nach Anspruch 1, nämlich {[N-(4,6-Dimethylpyridin-2-yl)-2-methioninamido]-carbonyl}-trimethylamino-bordihydrid, dessen N-Oxid, dessen Isomere sowie dessen Additionssalze mit einer pharmazeutisch annehmbaren Säure oder Base.

14. Verbindung nach Anspruch 1, nämlich N-(4,6-Dimethylpyridin-2-yl)-glycinamid sowie dessen N-Oxid und dessen Additionssalze mit einer pharmazeutisch annehmbaren Säure oder Base.

15. Verbindung nach Anspruch 1, nämlich N-(4,6-Dimethylpyridin-2-yl)-glycyl-glycinamid sowie dessen N-Oxid und dessen Additionssalze mit einer pharmazeutisch annehmbaren Säure oder Base.

16. Verbindung nach Anspruch 1, nämlich N-(4,6-Dimethylpyridin-2-yl)-glycyclglycylglycinamid sowie dessen N-Oxid und dessen Additionssalze mit einer pharmazeutisch annehmbaren Säure oder Base.

17. Verfahren zur Herstellung der Verbindungen der Formel (I) nach Anspruch 1, in der:
A eine Gruppe 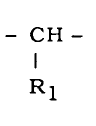 darstellt, dadurch gekennzeichnet, daß man als Ausgangsmaterial ein Derivat der Formel (II) verwendet: 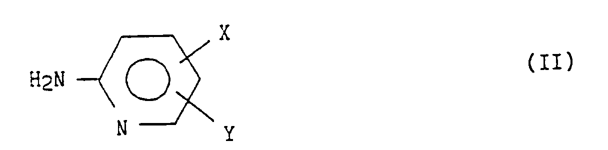
in der X und Y, die gleichartig oder verschieden sein können, eine Alkylgruppe darstellen,
welches man mit dem Derivat der Formel (III) kondensiert: 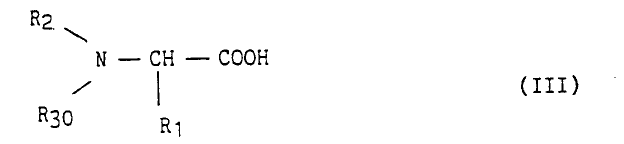
in der R₁ und R₂ die bezüglich der Formel (I) angegebenen Bedeutungen besitzen und R₃₀ eine tert.-Butoxycarbonylgruppe, Benzyloxycarbonylgruppe oder Fluorenylmethyloxycarbonylgruppe oder 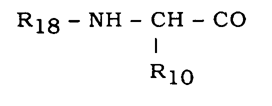 darstellt, worin R₁₀ die oben angegebenen Bedeutungen besitzt und R₁₈ eine tert.-Butoxycarbonylgruppe, Benzyloxycarbonylgruppe oder Fluorenylmethyloxycarbonylgruppe darstellt,
zur Bildung eines Derivats der Formel (IV): 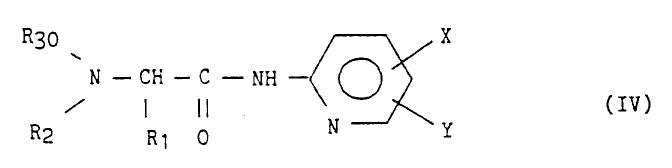 einem Sonderfall der Derivate der Formel (I), in der A eine Gruppe 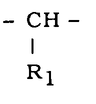 und R₃ eine Gruppe R₃₀ der oben definierten Art darstellen,
von welchem Derivat der Formel (IV) man anschließend die Schutzgruppe abspalten kann zur Bildung eines Derivats der Formel (V): 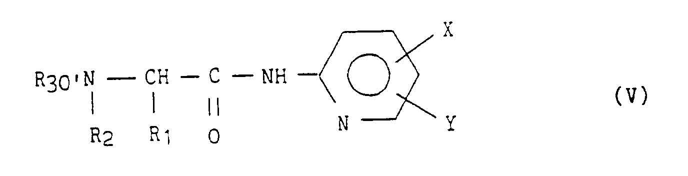
in der R₁, R₂, X und Y die oben angegebenen Bedeutungen besitzen und R_{30'} entweder ein Wasserstoffatom oder eine Gruppe 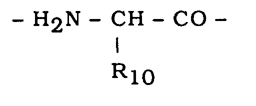
darstellt, in der R₁₀ die oben angegebenen Bedeutungen besitzt,
einem Sonderfall der Derivate der Formel (I), in der R₃ eine Gruppe R_{30'} und A eine Gruppe 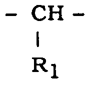 bedeuten,
worin R₁ und R_{30'} die oben angegebenen Bedeutungen besitzen,
welches Derivat der Formel (V) man, wenn R_{30'} ein Wasserstoffatom darstellt, mit einem Derivat der Formel (VI) kondensieren kann:
R₉COOH (VI)
in der R₉:
- eine Phenylgruppe,
- eine Phenylgruppe, die durch eine Alkylgruppe, Alkoxygruppe, Hydroxylgruppe, Trifluormethylgruppe oder ein Halogenatom substituiert ist,
- eine Gruppe 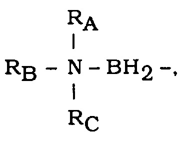 in der R_{A}, R_{B} und R_{C} die oben angegebenen Bedeutungen besitzen,
- eine Gruppe 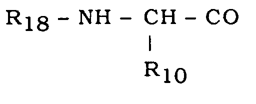
in der R₁₈ eine tert.-Butoxycarbonylgruppe, Benzyloxycarbonylgruppe, Fluorenylmethyloxycarbonylgruppe, Glycylgruppe, Glycylgruppe, die durch eine tert.-Butoxycarbonylgruppe, Benzyloxycarbonylgruppe oder Fluorenylmethyloxycarbonylgruppe substituiert ist, darstellt, und R₁₀ die oben angegebenen Bedeutungen besitzt, bedeutet, zur Bildung eines Derivats der Formel (VII): 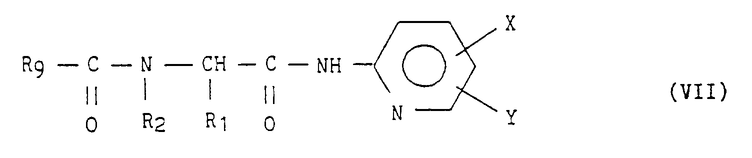
In der R, R₂, R₉, X und Y die oben angegebenen Bedeutungen besitzen, einem Sonderfall der Derivate der Formel (I), in der A eine Gruppe 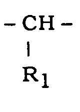 und R₃ eine Gruppe -CO-R₉ darstellen, worin R₁ und R₉ die oben angegebenen Bedeutungen besitzen,
welche man, wenn R₉ eine Gruppe 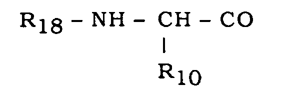 darstellt, gewünschtenfalls einer Behandlung zur Abspaltung der Schutzgruppen unterwerfen kann, zur Bildung eines Derivats der Formel (XVII): 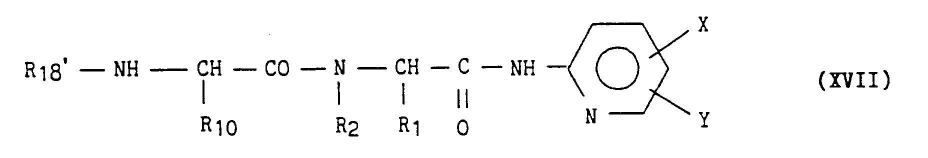
in der R_{18'} ein Wasserstoffatom oder eine Glycylgruppe darstellt und R₁, R₂, R₁₀, X und Y die oben angegebenen Bedeutungen besitzen,
einem Sonderfall der Derivate der Formel (I), in der A eine Gruppe 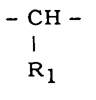 und und R₃ eine Gruppe 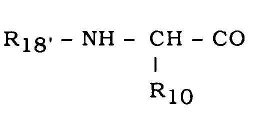 darstellen, worin R₁, R_{18'} und R₁₀ die oben angegebenen Bedeutungen besitzen,
welche Derivate der Formeln (IV), (V), (VII) und (XVII) man durch Behandeln mit Wasserstoffperoxid in die N-Oxide umwandelt und/oder man gegebenenfalls in die Isomeren auftrennt und gewünschtenfalls mit einer pharmazeutisch annehmbaren Säure oder Base in die Salze überführt.

18. Verfahren zur Herstellung der Verbindungen der Formel (I) nach Anspruch 1, worin A eine Gruppe (CH₂)ₙCO darstellt, in der n einen Wert zwischen 1 und 6 einschließlich aufweist, dadurch gekennzeichnet, daß man als Ausgangsmaterial ein Derivat der Formel (II) verwendet: 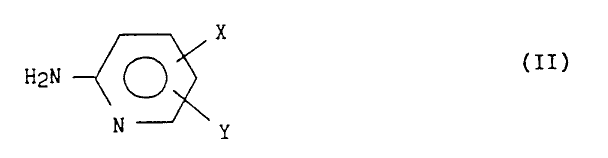
in der X und Y, die gleichartig oder verschieden sein können, jeweils eine Alkylgruppe darstellen,
welches man mit einem Derivat der Formel (VIII) kondensiert: 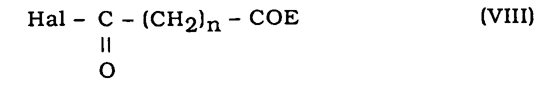
in der n die oben angegebene Bedeutung besitzt, Hal ein Halogenatom darstellt und E eine Alkoxygruppe bedeutet, zur Bildung eines Derivats der Formel (IX): 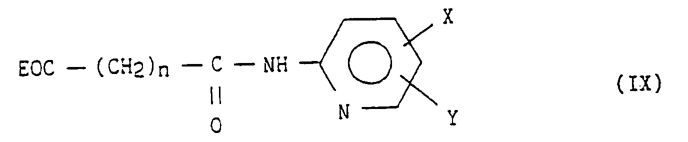
in der n, X, Y und E die oben angegebenen Bedeutungen besitzen,
welches bei der Behandlung mit einem alkalischen Mittel ein Derivat der Formel (X) ergibt: 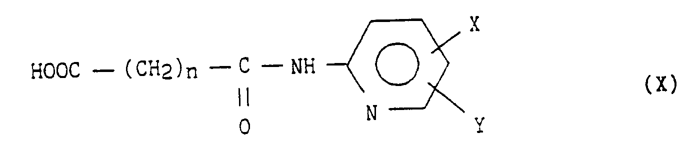
in der n, X und Y die oben angegebenen Bedeutungen besitzen,
welches man:
- entweder mit einem Derivat der Formel (XI) behandelt: 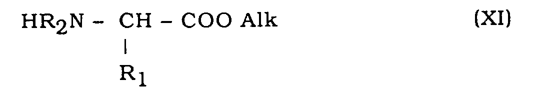
in der R₁ und R₂ die oben angegebenen Bedeutungen besitzen und Alk für eine Alkylgruppe steht,
zur Bildung eines Derivats der Formel (XII): 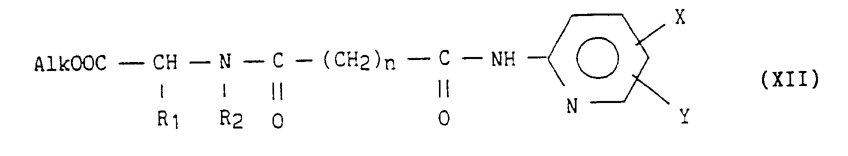 einem Sonderfall der Derivate der Formel (I), in der A eine Gruppe (CH₂)ₙCO und R₃ eine Gruppe 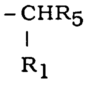 bedeuten, worin R₅ eine Alkoxycarbonylgruppe darstellt und n, R₁ und R₂ die oben angegebenen Bedeutungen besitzen,
welches Derivat der Formel (XII) durch Abspaltung der Schutzgruppe zu einem Derivat der Formel (XIII) führt: 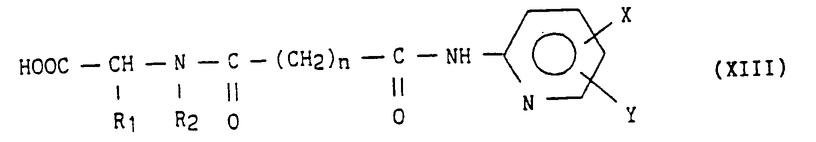 einem Sonderfall der Derivate der Formel (I), in der A eine Gruppe (CH₂)ₙCO und R₃ eine Gruppe 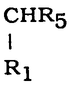 bedeuten, worin R₅ hier eine Carboxylgruppe darstellt und n, R₁ und R₂ die oben angegebenen Bedeutungen besitzen,
welches Derivat der Formel (XIII) man mit einem gegebenenfalls geschützten Derivat der Formel (XIV) behandeln kann: 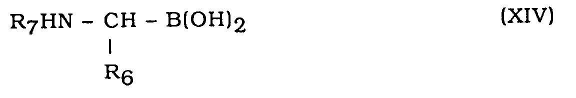 in der R₆ und R₇ die bezüglich der Formel (I) angegebenen Bedeutungen besitzen, so daß man nach einer eventuellen Abspaltung der Schutzgruppen ein Derivat der Formel (XV) erhält: 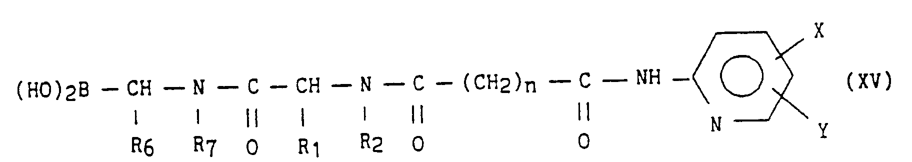 einem Sonderfall der Verbindungen der Formel (I), in der A eine Gruppe -(CH₂)ₙCO- und R₃ eine Gruppe 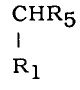 bedeuten, worin R₅ eine Gruppe 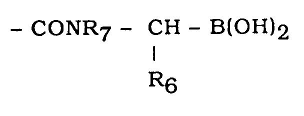 darstellt,
worin n, R₅, R₁, R₆ und R₇ die bezüglich der Formel (I) angegebenen Bedeutungen besitzen,
- oder direkt mit einem gegebenenfalls geschützten Derivat der Formel (XIV) umsetzt, so daß man nach der eventuellen Abspaltung der Schutzgruppe ein Derivat 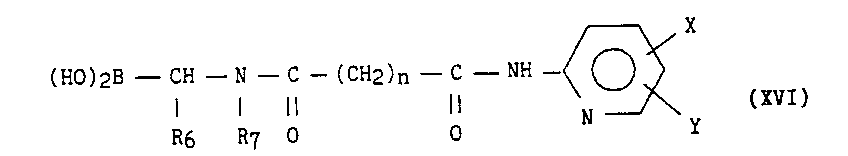 der Formel (XVI) erhält:
einem Sonderfall der Derivate der Formel (I), In der A eine Gruppe 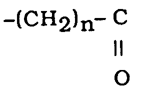 und R₃ eine Gruppe 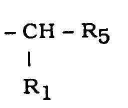 bedeuten, worin R₅ hier die Gruppe B(OH)₂ darstellt und R₆ die oben angegebenen Bedeutungen von R₁ aufweist und R₇ die oben für die Gruppe R₂ angegebenen Bedeutungen besitzt,
welche Derivate der Formeln (XII), (XIII), (XV) und (XVI) man durch Behandeln mit Wasserstoffperoxid In das N-Oxid umwandelt und/oder man gegebenenfalls In die Isomeren auftrennt und gewünschtenfalls mit Hilfe einer pharmazeutisch annehbmaren Säure oder Base in die Salze überführt.

19. Pharmazeutische Zubereitung enthaltend als Wirkstoff mindestens eine Verbindung nach einem der Ansprüche 1 bis 16 in Kombination mit einem oder mehreren inerten, nichttoxischen, pharmazeutisch annehmbaren Trägermaterialien oder Bindemitteln.

20. Pharmazeutische Zubereitung nach Anspruch 19 enthaltend mindestens einen Wirkstoff nach einem der Ansprüche 1 bis 16 zur Behandlung von Entzündungszuständen, der Psoriasis bei Hypercholesterinämien und Hypertriglyceridämien sowie der Psoriasis.
